# EUROPEAN PATENT APPLICATION

(11) **EP 2 267 117 A2**
(43) Date of publication of application: **29.12.2010**
(21) Application number: 10184571.7
(22) Date of filing: 27.06.2003
(51) Int. Cl.: C12N 5/077, A61K 38/18

(54) **Differentiation modulating agents and uses therefor**

(30) Priority: 27.06.2002 US 392130 P
(62) Divisional of application: 03735152.5
(71) Applicant: Verva Pharmaceuticals Pty Ltd, Melbourne 3004 (AU)
(72) Inventor: The designation of the inventor has not yet been filed
(74) Representative: Owen, Deborah Jane

(57) **Abstract**

This invention discloses methods and agents for modulating the differentiation potential and/or proliferation of preadipocytes. More particularly, the present invention discloses methods and agents for modulating a fibroblast growth factor (FGF) signaling pathway, especially the FGF-1 or FGF-2 signaling pathway, for treating or preventing adiposity-related conditions including, but not limited to, obesity, lipoma, lipomatosis, cachexia or lipodystrophy or the loss of adipose tissue in trauma or atrophic conditions

## Description

### FIELD OF THE INVENTION

THIS INVENTION relates generally to methods and agents for modulating the differentiation potential and/or proliferation of preadipocytes. More particularly, the present invention relates to fibroblast growth factor (FGF) signaling, especially FGF-1 and FGF-2 signaling, which causes the proliferation of preadipocytes and which potentiate preadipocytes to differentiate into adipocytes. Even more particularly, the invention relates to molecules that reduce, impair or abrogate FGF signaling, including antagonist molecules that are specific for *Fgf* or *Fgfr* polynucleotides or their expression products, and to the use of these molecules for the negative regulation of adipogenesis, including down-regulating the differentiation potential and/or proliferation of preadipocytes. The present invention also extends to the use of FGF or FGFR agonist molecules, including *Fgf* polynucleotides and FGF polypeptides, as well as their biologically active fragments, variants and derivatives, for increasing the differentiation potential and/or proliferation of preadipocytes. In addition, the present invention extends to methods of screening for agents that are useful for agonising or antagonising FGF signaling, including modulating the expression of a gene selected from a *Fgf* gene or a *Fgfr* gene or a gene belonging to the same biosynthetic or regulatory pathway as the *Fgf gene* or the *Fgfr* gene or for modulating the level or functional activity of an expression product of that gene. Furthermore, the invention relates to the use of such modulatory agents in methods for treating or preventing adiposity-related conditions including, but not limited to, obesity, lipoma, lipomatosis, cachexia or lipodystrophy or the loss of adipose tissue in trauma or atrophic conditions.

### BACKGROUND OF THE INVENTION

### OBESITY

Obesity represents a major health problem worldwide which is no longer confined to traditional 'Westernised' communities, as the high-fat diet and sedentary lifestyle of the traditional 'Western' countries is adopted in preference to traditional ethnic lifestyles (Doll el al. Int J Obes Relat Metab Disord 26(1): 48-57 2002) (Fall. Br Med Bull 60: 33-50 2001). The incidence of obesity and, in particular, obesity in children, is increasing at a faster rate than almost any other medical condition. Around 22 million children under the age of five years are overweight worldwide (Deckelbaum et al. Obes Res 9 Suppl 4: 239S-243S 2001), and over 7% of adults worldwide are obese with around a further 21% of adults being classified as overweight (Seidell Acta Paediatr Suppl 88(428): 46-50 1999). The World Health Organisation describes the high worldwide incidence of obesity in adults as a 'global pandemic'.

The association of obesity with serious co-morbidities such as cardiovascular diseases and type II diabetes (Fall 2001 *supra*) is the cause of its classification as a serious medical condition (James et al. Obes Res 9 Suppl 4: 228S-233S 2001). The consequential and significant financial impact of obesity on healthcare budgets has made obesity management and prevention a major priority for health promotion strategies. The aim of such strategies is weight reduction through caloric restriction and increased physical exercise, the premise of such goals being based on evidence that weight reduction in even morbidly obese individuals can lead to resolution or improvement of obesity-related pathologies (Melissas et al. Obest Surg 11(4): 475-81 2001).

Unfortunately, such strategies have met with limited success. The continuing increase in the obesity rate worldwide has forced a shift in focus of these obesity management and prevention strategies to metabolic and genetic therapeutic interventions. In order for such interventions to be successful, a detailed understanding of the cellular mechanisms of fat deposition is required.

Human adipose tissue is a dynamic organ with constant flux of both infra-cellular stored triglyceride and adipose cells throughout life. New adipocytes are formed by the proliferation and differentiation of preadipocytes, a process known as adipogenesis. Preadipocytes are fibroblast-like cells found in the stromo-vascular compartment of adipose tissue. Therapeutic interventions which inhibit adipogenesis would have profound clinical applications in the management of severely overweight patients.

Research has, thus far, discovered several protein, neuropeptide and transcriptional regulators of the cellular and molecular events underlying changes in adipose cell size or number. The effects of these substances indicate that adipocyte number and size are altered in a complex interplay involving hormonal and nutritional cues, which trigger downstream signaling *via* molecules which act in a cell-cell or cell-matrix manner (Gregoire Exp Biol Med (Maywood) 226(11): 997-1002 2001). The full repertoire of these molecules has yet to be established, as well as the way in which they interact and exert their effects on adipocytes.

Much has been learned about adipogenesis through development of techniques allowing the isolation and *in vitro* replication and differentiation of animal and human preadipocytes. Further insight has been gained by the study of murine preadipocyte cell lines (e,g., 3T3-L1) that differentiate *in vitro* to an adipocyte-like cell.

For human tissue, preadipocytes are isolated from adipose tissue using collagenase digestion and plated in serum-containing medium. Upon reaching confluence (with or without previous subculture) the cells are differentiated in a serum-free chemically and hormonally modified medium. This process is relatively inefficient, both in time and in the low percentage of cells that acquire a mature adipocyte phenotype.

The replication phase is enhanced by mitogens and insulin, and requires serum. The differentiation phase is completely inhibited by serum, and enhanced by insulin, corticosteroids, thyroid hormone and growth hormone. It has recently been shown that the thiazolidinedione (TZD) class of drugs stimulate differentiation *via* binding to PPARγ, a ligand-dependant transcription factor central to adipogenesis.

In work leading up to the present invention, an hypothesis was pursued that an interaction occurs between vascular cells and adipocytes. It is known that adipogenesis is preceded by the establishment of a fine vascular network (Hutley et al. Am J Physiol Endocrinol Metab 281(5): E1037-44 2001) and a paracrine interaction between preadipocytes and the endothelial cells of the microvasculature had been proposed (Hutley *et al. supra*) (Varzaneh et al. Metabolism 43(7): 906-12 1994). In an attempt to isolate candidate paracrine compounds, the present inventors co-cultured human pre-adipocytes with microvascular endothelial cells (MVEC) and found that acidic fibroblast growth factor (aFGF), also known as FGF-1, was present in the culture medium. Initial studies indicated unexpectedly that the source of this growth factor was primarily the MVEC and, contrary to previous studies, co-culturing preadipocytes with FGF-1 markedly promoted their growth and replication and also had a significant positive effect on the differentiation of the cells into mature adipocytes. Due to the potential functional redundancy between different members of the FGF family, it is believed that one or more other FGFs may also be associated with directly or indirectly modulating adipogenesis. Indeed, initial investigations indicate a pro-adipogenic effect for basic FGF (also known as FGF-2) that is similar to that shown by FGF-1.

### FGFS

The fibroblast growth factor family of structurally related polypeptide growth factors comprises over 20 members with protean recognised actions. There is limited direct coding sequence homology across the family. The name is misleading as stimulation of growth is not universal among family members but, as a family, the FGFs have critical roles in growth and development, cell replication and angiogenesis, cell survival and apoptosis, tumour development and morphogenesis. The FGFs belong to the larger Heparin-Binding Growth Factor family which comprises a large number of growth factors, some with similar or complementary actions to the FGFs.

FGFs are encoded by a number of different genes and have similar intron-exon organisation, with three coding regions in FGF-1-6. A central core region of 120 amino acids is highly conserved (70-100% identity) whilst other regions show marked diversity of sequence. FGFs vary in the presence of signal peptides or localisation sequences and in glycosylation sites and post-translational modification. Many of the FGFs show diversity with alternative promoter usage (eg FGF-1), alternative splicing (eg FGF-1 and -2) and the use of alternative polyadenylation sites (e.g., FGF-1 and -2). One mechanism of providing specificity of action is tissue-specific promoter usage (e.g., FGF-1).

All FGFs can be released from cells but some also accumulate in the nucleus or cytoplasm of producing and target cells. In addition, secreted FGFs are stored in the extracellular matrix and their further release is under protease control. FGFs are "released" from the extracellular matrix by one of two mechanisms. First, enzymatic cleavage of extracellular matrix components by proteases or heparinases results in release of FGF. Second, FGF can bind to a carrier protein (FGF-BP) that can in turn deliver FGF to its receptor. It is accepted that heparin or heparan-like glycosaminoglycans are essential for efficient FGF signalling. Tissue-specificity and/or differentiation stage-specificity of expression of some FGFs has been reported.

The association of the FGF family with components of the extracellular matrix is thought to serve two purposes: a) protection of FGFs from circulating protease degradation; and b) creation of a local reservoir of growth factor(s). The latter feature allows for strict spatial regulation of FGF signaling, as only cells in contact with the extracellular matrix are recipient to the FGF signal.

### FGF RECEPTORS

As with the ligands, the FGF receptors (FGFRs) comprise a gene family encoding five (at least) structurally related proteins. They are members of the tyrosine-kinase class of receptors and are widely expressed. Amino acid sequence of the five receptors is 60-95% with the best-conserved areas involved in signal transduction. FGFs have differing specificity in their binding to the receptors and this, along with cell-specific expression of the receptors and their splice variants, provides further diversity in signaling options. In addition to localisation in the plasma membrane, FGFRs are also expressed within the nuclear envelope and matrix. Signal transduction in response to FGFR occurs through receptor dimerisation and complex formation with heparan sulfate proteoglycans (HSPGs). Subsequent phosphorylation at multiple sites on the intracellular domain of the FGFR initiates recruitment and/or phosphorylation of multiple downstream signal transduction molecules and pathways. There are up to three characteristic Ig-like extracellular domains, placing the FGFRs into the IG super-receptor family (also contains PDGFR and IL-1R).

FGF signalling diversity is provided by cell specific expression of receptor combinations, cell specific expression of receptor isoform combinations, various hetero-dimer combinations and different repertoires of FGFs.

### HSPGs

HSPG are sulfated glycosaminoglycans covalently bound to a core protein that act to facilitate FGF-FGFR interaction. This may be due either to the HSPG inducing conformational changes in FGF and FGFR allowing each to dimerise and bind or due to the HSPG forming part of an active signaling complex with the FGF and FGFR. Experimental evidence to support both models exists, and it is highly conceivable that both mechanisms exist. Some FGF early responses may be elicited in the absence of HSPG but the latter appears essential for sustained signaling. HSPG also acts to protect FGFs from degradation in the extracellular matrix. HSPGs implicated to date in FGF signaling include the syndecans (cell-associated transmembrane proteoglycans), the glypicans (proteoglycans anchored to the plasma membrane by a glycosylphosphatidylinositol group) and perlecan (an extracellular, basal laminaproteoglycan). Evidence that the HSPGs are involved in the regulation of FGF signaling comes from in-vitro studies and studies of individuals with known HSPG mutations. These studies show, for example, that glypican can promote FGF-2-induced mitogenesis but inhibit FGF-7 responses.

### CYSTEINE-RICH FGFR

Cysteine-rich FGFR (CFR) is an integral membrane sialoglycoprotein that lacks heparan sulfate chains and binds FGFs. FGF binding to CFR and FGFR is mutually exclusive. CFR appears to have a role in FGF targeting to intracellular sites and in regulation of intracellular FGF concentrations.

### FGF SIGNALING PATHWAYS

### 1. FGFR-Dependent Intracellular Signaling

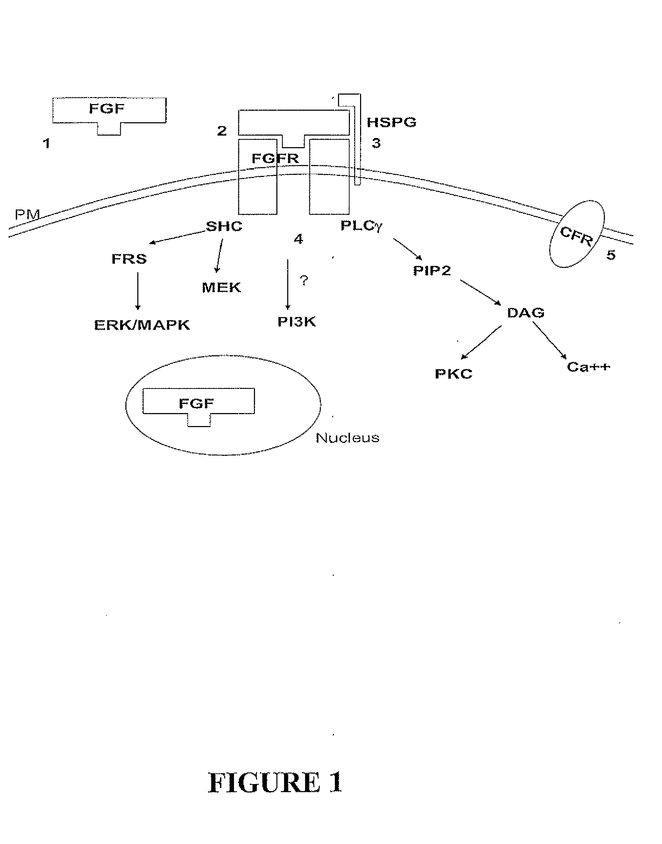

As outlined above, and with reference to the schematic representation of the FGF signaling pathway shown in Figure 1, ligand binding induces receptor dimerisation and autophosphorylation. Mutational analysis indicates that dimerisation alone is sufficient for signal transduction. FGFRs have a number of intracellular phosphorylation sites (seven in the case of FGFR-1) and phosphorylation site mutated, kinase dead, receptors are unable to transduce many biological signals of FGFs. However, some effects are retained, indicating that non receptormediated signaling pathways are an important consideration.

The signaling pathways known to be utilised by FGF/FGFR are (1) the SHC/FRS2-RAF/MAPKKK-MAPKK-MAPK pathway, and (2) the PLCγ□ □,PKC, Ca²⁺ pathway.

### a) SHC/FRS2-RAF/MAPKKK-MAPKK-MAPK pathway

Subsequent to receptor phosphorylation src homology (SH-2) domain-containing and phosphotyrosine-binding (PTB) domain proteins bind to specific intracellular FGFR phosphotyrosines. These proteins include PLCγ, SHC and FRS2 (FGFR substrate 2) and some of these molecules are specific to the FGFRs (e.g., FRS2) and others are more promiscuous (e.g., SHC). Upon phosphorylation, these docking proteins bind directly to the GRB2-SOS complex which functions as an adapter to RAS. Membrane-associated RAS then recruits and activates the MAPK transduction pathway.

It is noteworthy that each of the multiple kinases in the MAPK pathway are regulated by other signaling molecules downstream of FGF (and other) receptors, this "cross-talk" allowing much specificity of response.

### b) PLCγ, PKC, Ca⁺⁺ pathway

PLCγ is a SH-2 domain protein that binds to a specific phosphotyrosine in FGFRs (Y766 in FGFR-1) and subsequently hydrolyses phosphoinositol to inositol 1,4,5 triphosphate (IP₃) and diacyglycerol (DAG). IP₃ induces Ca²⁺ release from intracellular stores, whereas DAG activates PKC, a serine/threonine-specific Kinase.

Overall, the biological outcome of FGF stimulation depends on the quantities, combinations and subcellular localisation of FGFs, FGFRs, HSPGs and signaling intermediates found in the cell, in addition to modulation from other signaling molecules and pathways.

### 2. FGF Target Genes

FGF treatment alters expression of many genes, and can do so *via* non FGFR-mediated mechanisms. This is presumed to be a direct effect, and many FGFs have nuclear targeting motifs and are found in the nucleus, the nucleolus and in association with chromatin. The effect of FGFs on gene transcription is cell-type specific. Further, FGFs have been demonstrate to maintain the expression of genes whose initial induction is dependent on other factors. In addition to transcriptional regulation, FGFs also influence mRNA stability and translation and post-translational modification of proteins.

### 3. Interaction with other Growth Factor Signaling Pathways

FGFs can antagonise or synergise with many other growth factors. FGF co-operativity with transforming growth factor (TGF), insulin-like growth factor-1 (IGF-1) and WNT signaling is common.

From the foregoing, it is proposed, in accordance with the present invention, that molecules of a FGF signaling pathway, especially of the FGFR-1 or FGF-2 signaling pathway, can be used to provide both drug targets and regulators to promote or inhibit adipogenesis in *inter alia* adiposity-related conditions and also to provide diagnostic markers for predisposition to obesity, as described hereinafter.

### SUMMARY OF THE INVENTION

Accordingly, in one aspect, the present invention provides methods for modulating adipogenesis, which are useful *inter alia* in the treatment or prevention of adiposity-related conditions. These methods generally comprise contacting a cell with an agent for a time and under conditions sufficient to modulate a FGF signaling pathway. In some embodiments, the FGF signaling pathway is selected from the FGF-1 signaling pathway and the FGF-2 signaling pathway. Representative members of these pathways include, but are not limited to, FGFRs, HSPGs, members of the SHC/FRS2-RAF/MAPKKK-MAPKK-MAPK pathway, members of the PLCγ-PKC-Ca²⁺ pathway, members of the FGF-1 nuclear translocation pathway and intracellular binding partners such as P34 and FIF (FGF-interacting factor). Non limiting examples of suitable agents include small molecules, such as nucleic acids, peptides, polypeptides, peptidomimetics, carbohydrates, lipids or other organic (carbon containing) or inorganic molecules, as further described herein.

In some embodiments, the cell is contacted with an agent that modulates the expression of a gene or the level or functional activity of an expression product of the gene, wherein the gene is selected from a *Fgf* gene (e.g., *Fgf*-*1* or *Fgf*-*2*) and a gene belonging to the same regulatory or biosynthetic pathway as the *Fgf* gene (e.g., P34 and FIF). In these embodiments, the cell is suitably a microvascular endothelial cell, or precursor thereof.

In other embodiments, the cell is contacted with an agent that modulates the expression of a gene or the level or functional activity of an expression product of the gene, wherein the gene is selected from a *Fgfr* gene (e.g., *Fgfr-1, Fgfr-2, Fgfr-3, Fgfr-4, Fgfr-5,* especially *Fgfr*-*1*, *Fgfr-2, Fgfr*-*3, Fgfr-4*), a gene belonging to the same regulatory or biosynthetic pathway as the *Fgfr* gene (e.g., a gene involved in signaling *via* the Ras-Raf-MAPkinase pathway and/or *via* the phospholipase C pathway), a gene whose expression is modulated directly or indirectly by an expression product of the *Fgf* gene (e.g., PPARγ, IGFBP-3, IGFBP-6, IGF-2, IRS-2, PI3 kinase, PKCθ),or that agonises or antagonises the function of a FGFR with which a FGF (e.g., FGF-1 or FGF-2) interacts. In these embodiments, the cell is suitably a preadipocyte or precursor thereof.

In some embodiments, the agent reduces the expression of a gene (e.g., *Fgfr*-*1*, *Fgfr-2, Ppar*γ, *C*/*Ebp*α, *Plc*γ*2, Igfbp-3, Igfbp-6*) or the level or functional activity of an expression product of that gene (e.g., FGFR-1, FGFR,-2, PPARγ, *C*/EBPα*,* PLCγ2, IGFBP-3, IGFBP-6). In other embodiments, the agent increases the expression of a gene (e.g., *Fgf-1*, *Fgfr-3*, *Igf-2*, *Irs-2*, *Pi3 kinase, Pkc*θ) or the level or functional activity of an expression product of that gene (e.g., FGF-1, FGF-3, IGF-2, IRS-2, PI3 kinase, PKCθ). In still other embodiments, the agent antagonises the function of a FGFR, including reducing or abrogating the interaction between a FGFR and a FGF. In these embodiments, the agents antagonise a FGF signaling pathway and are therefore useful for directly or indirectly reducing or abrogating the differentiation potential and/or proliferation of a preadipocyte.

In some embodiments, the agent reduces the expression of a gene (e.g., *Fgf-1, Igf*-*2*, *Irs*-*2*, *Pi3 kinase, Pkc*θ) or the level or functional activity of an expression product of that gene (e.g., FGF-1, IGF-2, IRS-2, PI3 kinase, PKCθ). In other embodiments, the agent increases the expression of a gene (e.g., *Fgfr-1, Fgfr-2, Ppar*γ*, C*/*Ebp*α, *Plcy2, Igfbp-3, Igfbp-6*) or the level or functional activity of an expression product of that gene (e.g., FGFR-1, FGFR-2, PPARγ, C/EBPα, PLCγ2, IGFBP-3, IGFBP-6). In still other embodiments, the agent agonises the function of a FGFR, including enhancing, promoting or otherwise capacitating the interaction between a FGFR and a FGF. In these embodiments, the agents agonise a FGF signaling pathway and are useful therefore for directly or indirectly increasing the differentiation potential and/or proliferation of a preadipocyte.

Suitably, the agent increases or reduces the expression of the gene or the level or functional activity of an expression product of that gene by at least 10%, 20%, 30%, 40%, 50%, 60%, 70%, 80%, 90% relative to the expression, level or functional activity in the absence of the agent.

In yet another aspect, the invention provides methods for identifying agents that modulate a FGF signaling pathway: These methods typically comprise contacting a preparation with a test agent, wherein the preparation comprises (i) a polypeptide comprising an amino acid sequence corresponding to at least a biologically active fragment of a polypeptide component of the FGF signaling pathway, or to a variant or derivative thereof; or (ii) a polynucleotide comprising at least a portion of a genetic sequence that regulates the component, which is operably linked to a reporter gene. A detected change in the level and/or functional activity of the polypeptide component, or an expression product of the reported gene, relative to a normal or reference level and/or functional activity in the absence of the test agent, indicates that the agent modulates the FGF signaling pathway.

Still another aspect of the present invention provides methods for identifying agents that modulate a FGF signaling pathway. These methods generally comprise contacting a first sample of cells expressing a FGFR with a FGF and measuring a marker; contacting a second sample of cells expressing the FGFR with an agent and the FGF, and measuring the marker; and comparing the marker of the first sample of cells with the marker of the second sample of cells. In various embodiments, these methods measure the levels of various markers (e.g., glycerol 3-phosphate dehydrogenase; G3PDH, and intracellular components of the FGF pathway), or combinations of markers, associated with the proliferation and/or differentiation of preadipocytes.

In accordance with the present invention, the agents broadly described above are useful for modulating adipogenesis in adiposity-related conditions. The adiposity-related conditions include, but are not restricted to, obesity, lipoma, lipomatosis, cachexia or lipodystrophy or the loss of adipose tissue in trauma or atrophic conditions. Thus, another aspect of the present invention contemplates the use of an agent, which is optionally formulated with a pharmaceutically acceptable carrier or diluent, for inhibiting or decreasing adipogenesis, or for controlling adipogenesis in obesity or in conditions of localised, abnormal increases in adipogenesis, wherein the agent antagonises a FGF signaling pathway as broadly described above.

In yet another aspect, the present invention resides in the use of an agent, which is optionally formulated with a pharmaceutically acceptable carrier or diluent, for stimulating adipogenesis in the treatment or prophylaxis of cachexia or in conditions of localised deficiencies in adiposity, wherein the agent agonises a FGF signaling pathway as broadly described above.

The agent used in the above methods is **characterised in that** it binds to an expression product of a gene as broadly described above or to a genetic sequence (e.g., a transcriptional element) that modulates the expression of the gene, as determined by: contacting a preparation comprising at least a portion of an expression product of a gene as broadly described above, or a variant or derivative of the expression product, or a genetic sequence that modulates the expression of the gene, with the agent; and detecting a change in the level or functional activity of the at least a portion of the expression product, or the variant or derivative, or of a product expressed from the genetic sequence.

In some embodiments, an agent which inhibits or otherwise decreases adipogenesis binds to a FGF or FGFR or to a genetic sequence (e.g., a transcriptional element) that modulates the expression of a *Fgf* or *Fgfr* gene, as determined by: contacting a preparation comprising a FGF or FGFR polypeptide or biologically active fragment thereof, or variant or derivative of these, or a genetic sequence that modulates the expression of a *Fgf* or *Fgfr* gene; and detecting a decrease in the level or functional activity of the FGF or FGFR polypeptide or biologically active fragment thereof, or variant or derivative, or of a product expressed from the genetic sequence.

In other embodiments, an agent which inhibits or otherwise decreases adipogenesis antagonises a FGF signaling pathway, as determined by: contacting a FGFR and a FGF with the agent and measuring the binding of the FGFR with the FGF. In these embodiments, agents can bind to FGF or FGFR and test positive when they reduce or abrogate the binding of the FGFR with the FGF. The agents can be small molecules or antigen-binding molecules specific for the FGF or the FGFR.

In other embodiments, an agent which inhibits or otherwise decreases adipogenesis antagonises a FGF signaling pathway, as determined by: contacting a FGFR and an HSFG with the agent and measuring the binding of the FGFR with the HSPG. In these embodiments, agents can bind to FGF or HSPG and test positive when they reduce or abrogate the binding of the HSPG with the FGFR. The compounds can be small molecules or antigen-binding molecules specific for the FGFR or the HSPG.

In other embodiments, an agent which inhibits or otherwise decreases adipogenesis antagonises a FGF signaling pathway, as determined by: contacting a FGF and a CFR with the agent and measuring the binding of the FGF with the CFR. In these embodiments, agents can bind to FGF or CFR and test positive when they reduce or abrogate the binding of the FGF with the CFR. The compounds can be small molecules or antigen-binding molecules specific for the FGF or the CFR.

In still other embodiments, an agent which inhibits or otherwise decreases adipogenesis antagonises a FGF signaling pathway, as determined by: contacting a first sample of cells selected from preadipocytes or their precursors with a FGF and measuring differentiation and/or proliferation of the cells; contacting a second sample of cells selected from preadipocytes or their precursors with an agent and the FGF, and measuring differentiation and/or proliferation of the cells; comparing the differentiation and/or proliferation of the first sample of cells with the differentiation and/or proliferation of the second sample of cells. In these embodiments, the agents antagonise the FGF signaling pathway by interfering with the association of the FGF and a FGFR, by interfering with the phosphorylation of a FGFR, by interfering with components of the signaling pathway upstream or downstream of the FGF/FGFR interaction, by interfering with the association of a FGFR with an HSPG, by interfering with the association of the FGF and CFR, or by interfering with the dimerisation of a FGFR. In some embodiments, agents that antagonise the FGF signaling pathway interfere with a signaling pathway selected from the TGF, IGF-1 and WNT signaling pathways.

In further embodiments, an agent which inhibits or otherwise decreases adipogenesis antagonises a FGF signaling pathway, as determined by: administering to an animal model, or a human, an agent that antagonises the signaling pathway, and measuring the animal's responsiveness to the agent. In these embodiments, the method can be practiced with agents as described above and animals can be examined for inhibition or reduction of adipogenesis in obesity or in conditions of localised, abnormal increases in adipogenesis.

In still other embodiments, an agent which stimulates adipogenesis binds to a FGFR or to a genetic sequence (e.g., a transcriptional element) that modulates the expression of a *Fgfr* gene as determined by: contacting a preparation comprising a FGFR polypeptide or biologically active fragment thereof, or variant or derivative of these, or a genetic sequence that modulates the expression of a *Fgf* or *Fgfr* gene; and detecting an increase in the level or functional activity of the FGFR polypeptide or biologically active fragment thereof, or variant or derivative, or of a product expressed from the genetic sequence.

In other embodiments, an agent which stimulates adipogenesis agonises a FGF signaling pathway, as determined by: contacting a FGFR and a FGF with the agent and measuring the binding of the FGFR with the FGF. In these embodiments, agents can bind to FGF or FGFR and test positive when they stimulate the FGFR interaction with the FGF. The agents can be small molecules or antigen-binding molecules specific for the FGF or the FGFR.

In other embodiments, an agent which stimulates adipogenesis agonises a FGF signaling pathway, as determined by: contacting a FGFR and an HSPG with the agent and measuring the binding of the FGFR with the HSPG, In these embodiments, agents can bind to FGF or HSPG and test positive when they stimulate the HSPG interaction with the FGFR. The compounds can be small molecules or antigen-binding molecules specific for the FGF or the HSPG.

In other embodiments, an agent which stimulates adipogenesis agonises a FGF signaling pathway, as determined by: contacting a FGF and a CFR with the agent and measuring the binding of the FGF with the CFR. In these embodiments, agents can bind to FGF or CFR and test positive when they stimulate the CFR interaction with the FGF. The compounds can be small molecules or antigen-binding molecules specific for the FGF or the CFR.

In still other embodiments, an agent which enhances adipogenesis agonises a FGF signaling pathway, as determined by: contacting a first sample of cells selected from preadipocytes or their precursors with a FGF and measuring differentiation and/or proliferation of the cells; contacting a second sample of cells selected from preadipocytes or their precursors with an agent and the FGF, and measuring differentiation and/or proliferation of the cells; comparing the differentiation and/or proliferation of the first sample of cells with the differentiation and/or proliferation of the second sample of cells, In these embodiments, compounds agonise the FGF signaling pathway by stimulating the association of the FGF with a FGFR, by stimulating the phosphorylation of a FGFR, by stimulating the association of a FGFR with an HSPG, by stimulating the association of FGF and CFR, by stimulating the dimerisation of a FGFR or by stimulating the signaling pathway upstream or downstream of the FGF/FGFR interaction.

In still other embodiments, an agent which stimulates adipogenesis agonises a FGF signaling pathway, as determined by: administering to an animal model, or a human, an agent that agonises the signaling pathway, and measuring the animal's responsiveness to the agent. In these embodiments, the method can be practiced with agents as described above and animals can be examined for stimulating adipogenesis in the treatment or prophylaxis of cachexia or in conditions of localised deficiencies in adiposity.

Still another aspect of the present invention provides methods of producing an agent for modulating adipogenesis in adiposity-related conditions. These methods generally comprise: testing an agent suspected of modulating a FGF signaling pathway as broadly described above; and synthesising the agent on the basis that it tests positive for the modulation. Suitably, the method further comprises derivatising the agent, and optionally formulating the derivatised agent with a pharmaceutically acceptable carrier and/or diluent, to improve the efficacy of the agent for treating or preventing the adiposity-related condition(s).

According to another aspect, the present invention provides methods for detecting the presence or diagnosing the risk of an adiposity-related condition in a patient. These methods generally comprise determining the presence of an aberrant gene involved in the FGF signaling pathway or of an aberrant expression product of a gene involved in the FGF signaling pathway in a biological sample obtained from the patient, wherein the aberrant gene or the aberrant expression product correlates with the presence or risk of the condition.

In some embodiments, the aberrant gene is selected from an aberrant *Fgf* gene and an aberrant *Fgfr* gene. In other embodiments, the aberrant expression product is selected from an aberrant *Fgf* expression product and an aberrant *Fgfr* expression product.

In yet another aspect, the present invention encompasses methods for detecting the presence or diagnosing the risk of a condition associated with aberrantly increased adiposity in a patient. These methods generally comprise determining the presence of an aberrant gene involved in the FGF signaling pathway or of an aberrant expression product of a gene involved in the FGF signaling pathway in a biological sample obtained from the patient, wherein the aberrant gene or the aberrant expression product correlates with the presence or risk of the condition. Conditions associated with aberrantly increased adiposity include, but are not limited to, obesity or conditions of localised, abnormal increases in adipogenesis such as lipoma and lipomatosis.

Another aspect of the present invention provides methods for detecting the presence or diagnosing the risk of a condition associated with aberrantly increased adiposity in a patient. These methods generally comprise determining in a cell a level or functional activity of an expression product of a gene involved in the FGF signaling pathway, which is different than a normal (e.g., non-obese) reference level or functional activity of the expression product. In some embodiments, the method comprises determining an increase or elevation in the level or functional activity of the expression product of a gene selected from *Fgfr-1, Fgfr-2*, *Ppar*γ, *C*/*Ebp*α*, Plc*γ*2*, *Igfbp-3* and *Igfbp-6.* In other embodiments, the method comprises determining a decrease in the level or functional activity of the expression product of a gene selected from *Fgf-1*, *Fgfr-3*, *Igf-2, Irs-2*, *Pi3 kinase* and *Pkc*θ. In these embodiments, the cell is a preadipocyte or precursor thereof.

In other embodiments, the method comprises determining an increase or elevation in the level or functional activity of the expression product of a gene selected from *Fgf*-*1* and *Fgf*-*2.* In these embodiments, the cell is a microvascular endothelial cell.

Another aspect of the present invention contemplates methods for inhibiting or reducing adipogenesis in obesity or in conditions of localised, abnormal increases in adipogenesis. These methods generally comprise administering to a patient in need of such treatment an adipogenesis-inhibiting effective amount of an agent which impairs or interferes with a FGF signaling pathway as broadly described above, and optionally a pharmaceutically acceptable carrier or diluent.

Yet another aspect of the present invention contemplates methods for treatment or prophylaxis of cachexia or conditions of localised deficiencies in adiposity. These methods generally comprise administering to a patient in need of such treatment an adipogenesis-enhancing effective amount of an agent which stimulates a FGF signaling pathway as broadly described above, and optionally a pharmaceutically acceptable carrier or diluent.

Still another aspect of the present invention provides the use of an agent as broadly described above in the preparation of a medicament for treating or preventing an adiposity-related condition.

### BRIEF DESCRIPTION OF THE DRAWINGS

Figure 1 is a schematic representation of the FGF signaling pathway.

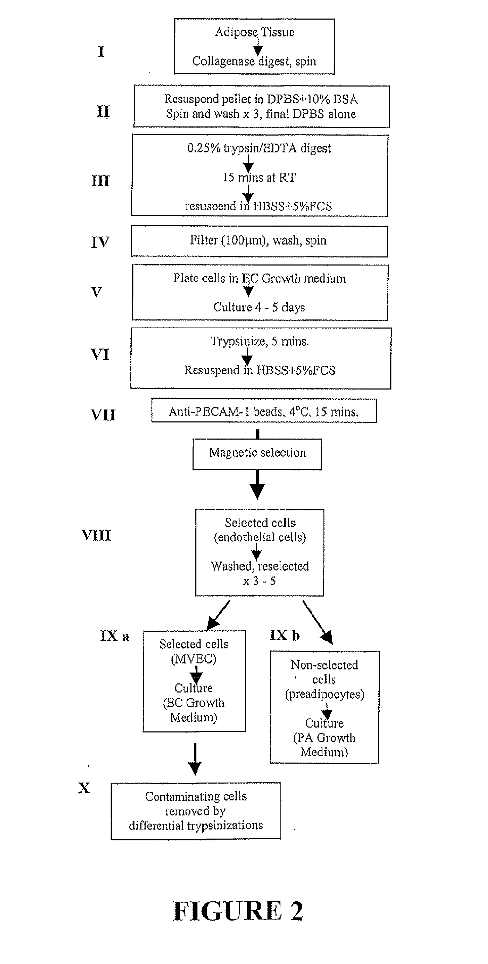

Figure 2 is a schematic representation of the method for isolation and separation of microvascular endothelial cells (MVEC) and preadipocytes (PA) from human adipose tissue. DPBS: deionised phosphate buffered saline; RT: room temperature; HBSS: Hank's balanced salt solution; FCS: fetal calf serum; EC: endothelial cells; PECAM-1: platelet-endothelial cell adhesion molecule 1.

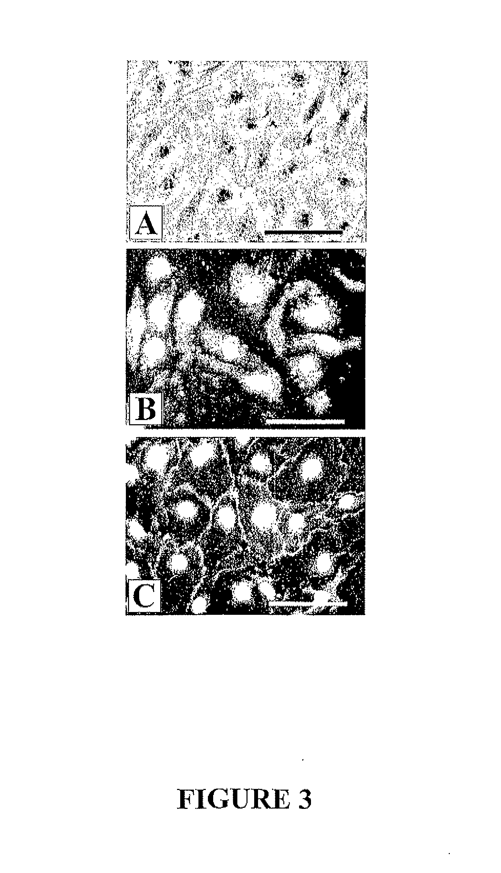

Figure 3 is a photographic representation illustrating the morphology of adipose tissuederived MVEC. A: phase-contrast photomicrograph of MVEC isolated from human adipose tissue. Note the typical cobblestone morphology and the prominent, centrally located nuclei. B: immunocytochemical staining for von Willebrand's factor (vWF) shows prominent perinuclear cytoplasmic staining. C: immunocytochemical staining for PECAM-1 shows junctional staining consistent with plasma membrane expression. In B and C, nuclei counterstained with propidium iodide. (Bar = 10 µm; original magnification x200).

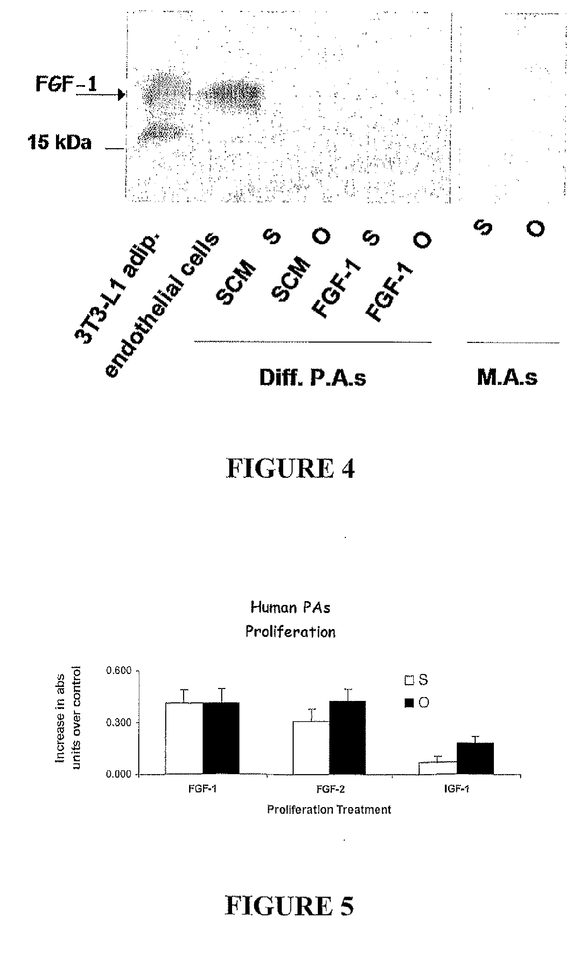

Figure 4 is a photographic representation of a Western blot analysis, showing strong expression of FGF-1 in adipose-derived MVEC and also in 3T3-L1 adipocytes (expression was also shown in 3T3-L1 fibroblasts). FGF-1 protein is undetectable in both human preadipocytes (+/- exposure to FGF-1 and adipocytes. RT-PCR analysis corroborated these expression patterns.
Figure 5 is a graphical representation showing a marked increase in proliferation of human preadipocytes (PAs) in response to both FGF-1 and FGF-2 (with FGF-1 effects on proliferation greater than FGF-2).

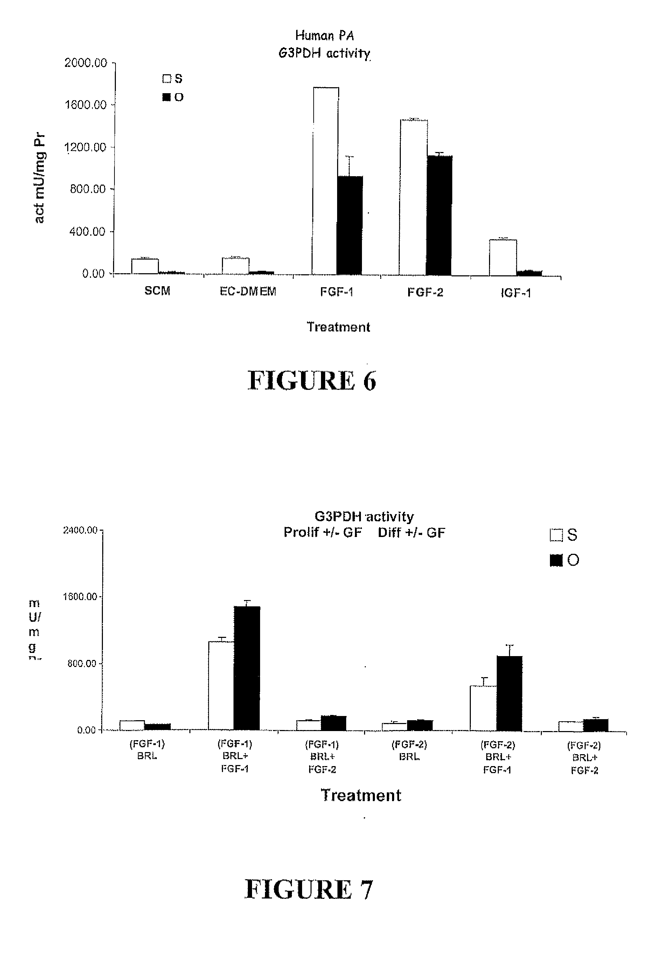

Figure 6 is a graphical representation showing a marked increase in differentiation of human preadipocytes (PAs) in response to both FGF-1 and FGF-2, (with FGF-1 effects on differentiation greater than FGF-2).
Figure 7 is a graphical representation showing the effects of combination treatments of FGF-1 and FGF-2. The results show that both FGF-1 and FGF-2 were adipogenic if present either during replication or during differentiation and that the adipogenic effect of FGF-1 during replication and differentiation are independent and additive. BRL = rosiglitazone; brackets denote replication treatment.

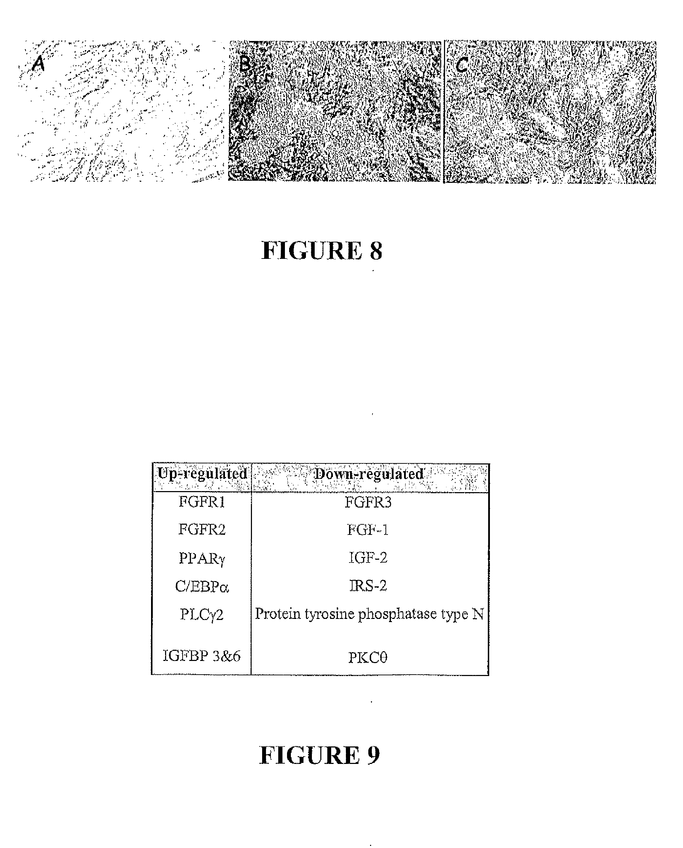

Figure 8 is a photographic representation showing the differentiation of human preadipocytes (PAs) using a 3T3-L1 differentiation protocol that utilises serum-containing medium (SCM) (+ insulin and, for the first 3 days, dexamethasone and rosiglitazone). Panel (A) shows PAs that have not been exposed to FGF-1 during proliferation prior to differentiation. Panels (B) and (C) show subcutaneous & omental PAs, respectively, that have been proliferated for six weeks in the presence of FGF-1 and subsequently differentiated in SCM. This is the first report of human PAs differentiating in the presence of serum. (Bar = 10 µm)
Figure 9 is a tabular representation showing the results of two separate gene array experiments which compared gene expression in human PAs grown to confluence in serum-containing medium in the presence and absence of FGF-1. Gene expression was considered to be influenced by FGF-1 if expression was consistently (CV<5%) increased or reduced by at least 50%.

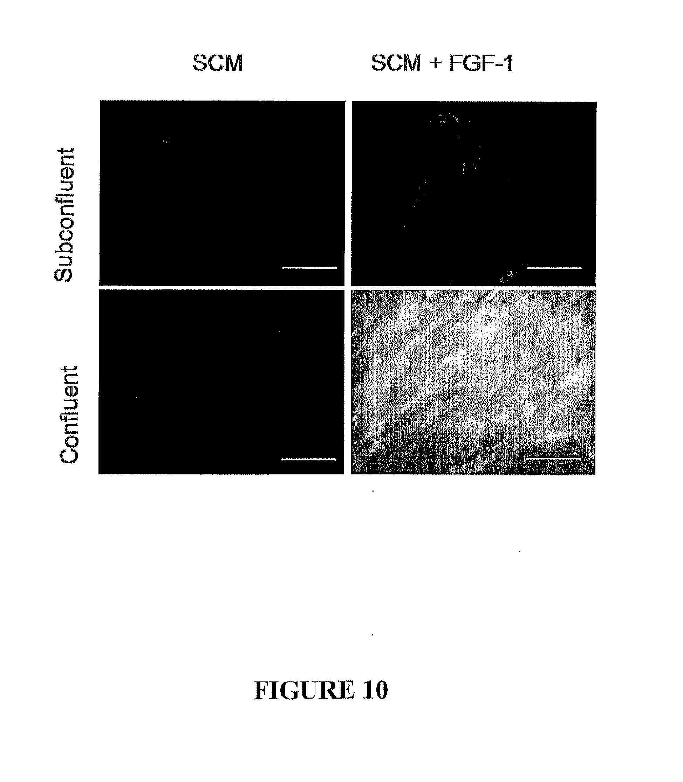

Figure 10 is a photographic representation showing that PLCγ2 is an intracellular molecule important in FGFR signal transduction. Both Western blot analysis and immunofluorescence confirmed that expression of this molecule is increased in human PAs grown to confluence in the presence of FGF-1 *cf.* cells that have not been exposed to this growth factor. The immunofluorescence data also show that PLC γ2 expression is greatly unregulated at confluence- the stage at which induction of differentiation occurs. (Bar = 10 µm)

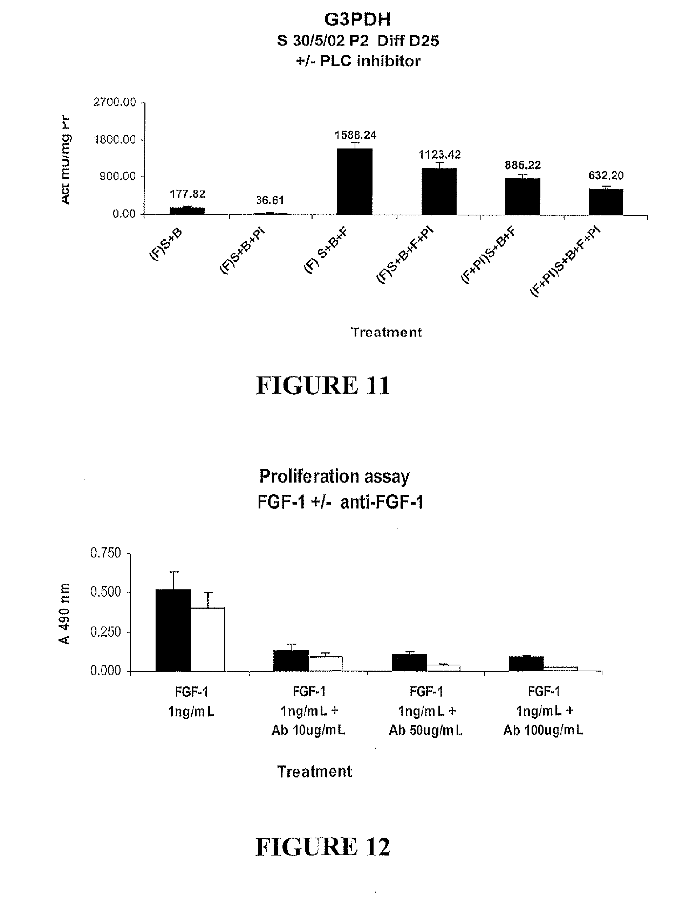

Figure 11 is a graphical representation showing that inhibition of PLCγ2 markedly reduces FGF-1 induced differentiation of preadipocytes.
Figure 12 is a graphical representation showing that neutralising anti-FGF-1 antibody abrogates FGF-1-induced human preadipocyte replication.

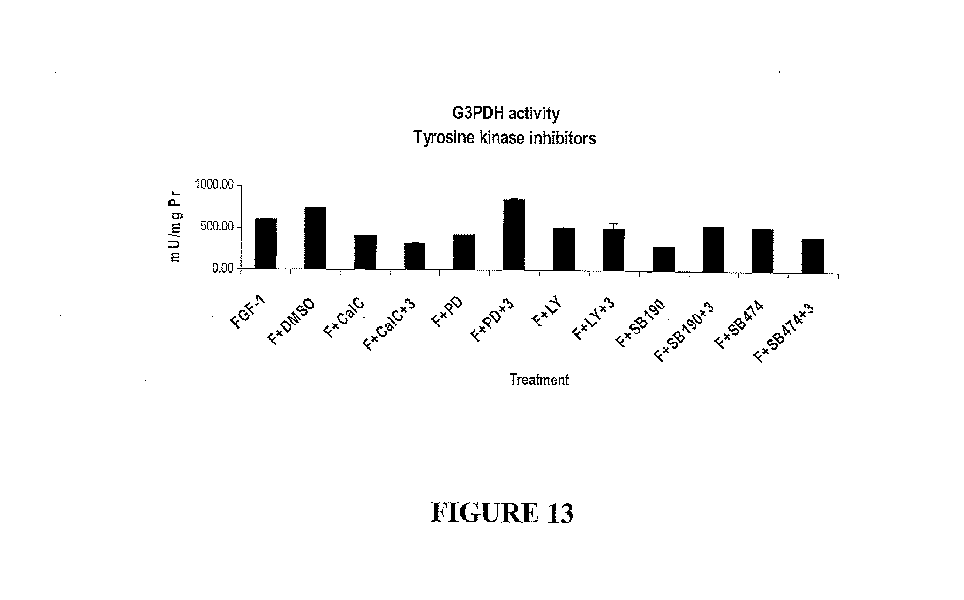

Figure 13 is a graphical representation showing that inhibition of post FGFR signal transduction pathways has marked effects on FGF-1-mediated human adipogenesis.

### DETAILED DESCRIPTION OF THE INVENTION

### 1. Definitions

Unless defined otherwise, all technical and scientific terms used herein have the same meaning as commonly understood by those of ordinary skill in the art to which the invention belongs. Although any methods and materials similar or equivalent to those described herein can be used in the practice or testing of the present invention, preferred methods and materials are described. For the purposes of the present invention, the following terms are defined below.

The articles "a" and "an" are used herein to refer to one or more than one (ie to at least one) of the grammatical object of the article. By way of example, "an element" means one element or more than one element.

By "*a gene belonging to the same regulatory or biosynthetic pathway"* is meant a gene whose expression product can modulate or otherwise influence FGF or FGFR protein levels and/or *Fgf or Fgfr* transcription levels. For example, a gene belonging to the same regulatory pathway as *Fgf* may encode an upstream regulator of *Fgf*/FGF, or a downstream regulatory target of *Fgf*/FGF, instead of *Fgf*/FGF. Alternatively, a gene belonging to the same regulatory or biosynthetic pathway as a *Fgfr* gene includes genes which directly or indirectly modulate the expression of a *Fgfr* gene as well as genes which act as signal transducers for FGFR activation. Such signaling molecules are involved in communicating and/or mediating the effects of FGFR activation and are commonly known in the art. They include *inter alia* molecules involved in the phospholipase C (PLC)-γ, Crk, SNT-1/FRS2 and/or Src signaling pathways.

The term *"aberrant polynucleotide"* as used herein refers to a polynucleotide which is distinguished from a "normal" reference polynucleotide by the substitution, deletion or addition of at least one nucleotide and which correlates with the presence or risk of adipogenic defects including an elevated rate of adipogenesis compared to a non-obese, reference value.

The term *"aberrant polypeptide"* refers to a polypeptide which is distinguished from a "normal" reference polypeptide by the substitution, deletion or addition of at least one amino acid residue and which correlates with the presence or risk of adipogenic defects including an elevated rate of adipogenesis compared to a non-obese, reference value.

*"Amplification product"* refers to a nucleic acid product generated by a nucleic acid amplification technique.

By *"antigen-binding molecule"* is meant a molecule that has binding affinity for a target antigen. It will be understood that this term extends to immunoglobulins, immunoglobulin fragments and non-immunoglobulin derived protein frameworks that exhibit antigen-binding activity.

*"Antigenic or immunogenic activity"* refers to the ability of a polypeptide, fragment, variant or derivative according to the invention to produce an antigenic or immunogenic response in an animal, suitably a mammal, to which it is administered, wherein the response includes the production of elements which specifically bind the polypeptide or fragment thereof.

By *"biologically active fragment"* is meant a fragment of a full-length parent polypeptide which fragment retains an activity of the parent polypeptide. As used herein, the term *"biologically active fragment*" includes deletion variants and small peptides, for example of at least 6, 7, 8, 9, 10, 11, 12, 13, 14, 15, 16, 17, 18, 19, 20, 21, 22, 23, 24, 25, 26, 27, 28, 29, 30, 35, 40, 45, 50 contiguous amino acid residues, which comprise an activity of the parent polypeptide. Peptides of this type may be obtained through the application of standard recombinant nucleic acid techniques or synthesised using conventional liquid or solid phase synthesis techniques. For example, reference may be made to solution synthesis or solid phase synthesis as described, for example, in Chapter 9 entitled *"Peptide Synthesis"* by Atherton and Shephard which is included in a publication entitled *"Synthetic Vaccines"* edited by Nicholson and published by Blackwell Scientific Publications. Alternatively, peptides can be produced by digestion of a polypeptide of the invention with proteinases such as endoLys-C, endoArg-C, endoGlu-C and staphylococcus V8-protease. The digested fragments can be purified by, for example, high performance liquid chromatographic (HPLC) techniques,

The term *"biological sample"* as used herein refers to a sample that may extracted, untreated, treated, diluted or concentrated from a patient. Suitably, the biological sample is a tissue biopsy, more preferably from subcutaneous or omental tissue biopsy.

By *"cachexia"* is meant a clinical state of below-normal adiposity which may or may not be accompanied by malnutrition or general ill-health and which may be secondary to one or more other pathologies. The term cachexia extends to but is not limited by the following conditions: cancerous cachexia, fluoric cachexia, hypophysial cachexia, cachexia hypophysiopriva, malarial cachexia, cachexia mercurialis, pituitary cachexia, saturnine cachexia, cachexia suprarenalis and uraemic cachexia or conditions of localised deficiencies in adiposity.

Throughout this specification, unless the context requires otherwise, the words *"comprise", "comprises"* and *"comprising"* will be understood to imply the inclusion of a stated step or element or group of steps or elements but not the exclusion of any other step or element or group of steps or elements.

The phrase *"conditions of localised, abnormal increases in adipogenesis*" as used herein includes pathologies characterised by and/or associated with anatomically localised disregulated adipogenesis that lead to circumscribed depositions of fat tissue. Such conditions include but are not limited to lipoma and lipomatosis.

By "*conditions of localised deficiencies in adiposity*" is meant anatomically restricted inadequacies in adipose tissue, which be caused by *inter alia* traumatic bodily injury which results in loss of subcutaneous adipose tissue, heat or chemical burns, lipodystrophy or atrophic conditions. The term "*lipodystrophy*" as used herein refers to any pathological conditions associated with or characterised by disturbances in fat metabolism resulting in an absence of subcutaneous fat which may congenital or acquired and partial or total. Such conditions include *inter alia* congenital generalised lipodystrophy, congenital progressive lipodystrophy, generalised lipodystrophy, Whipple's disease, partial lipodystrophy, progressive lipodystrophy and total lipodystrophy. The term "*atrophic conditions*" as used herein is meant a condition associated with and/or characterised by a reduction or wasting of body tissue including adipose tissue, which may or may not be anatomically localised, the cause of which may include *inter alia* damage to the central and/or peripheral nervous systems, inactivity and/or incapacitation. Such atrophic conditions include but are not limited to serous atrophy, spinal muscular atrophy, arthritic atrophy, compression atrophy, neuropathic atrophy, atrophy of disuse, endocrine atrophy and senile atrophy.

By "*corresponds to*" or "*corresponding to*" is meant (a) a polynucleotide having a nucleotide sequence that is substantially identical or complementary to all or a portion of a reference polynucleotide sequence or encoding an amino acid sequence identical to an amino acid sequence in a peptide or protein; or (b) a peptide or polypeptide having an amino acid sequence that is substantially identical to a sequence of amino acids in a reference peptide or protein.

By "*derivative*" is meant a polypeptide that has been derived from the basic sequence by modification, for example by conjugation or complexing with other chemical moieties or by post-translational modification techniques as would be understood in the art. The term "*derivative*" also includes within its scope alterations that have been made to a parent sequence including additions or deletions that provide for functional equivalent molecules.

The term "*differentiation potential*" as used herein means the capacity of a preadipocyte to respond, or the magnitude of the response, to a signal which promotes its functional maturation into an adipocyte. An "increase in differentiation potential" may be seen to be conferred by a test molecule wherein, for example, a co-culture of preadipocytes with the test molecule for a sufficient time and under appropriate conditions results in an increase in the response of the preadipocytes to a differentiation-inducing agent, which may be observed *inter alia* as a rise in the number of preadipocytes undergoing differentiation or an increase in the rate at which the preadipocytes undergo differentiation.

By "*effective amount*", in the context of modulating an activity or of treating or preventing a condition is meant the administration of that amount of active ingredient to an individual in need of such modulation, treatment or prophylaxis, either in a single dose or as part of a series, that is effective for modulation of that effect or for treatment or prophylaxis or improvement of that condition. Non-limiting examples of such improvements in an individual suffering conditions of localised, abnormal increases in adipogenesis include reduced fat deposits, increased leanness, weight loss and an improvement in the symptoms relating to cardiovascular disease and diabetes. Non-limiting examples of improvements for an individual suffering cachexia and conditions of localised deficiencies in adiposity include enhanced fat deposits, weight gain and improvement in the symptoms relating to atrophic conditions. The effective amount will vary depending upon the health and physical condition of the individual to be treated, the taxonomic group of individual to be treated, the formulation of the composition, the assessment of the medical situation, and other relevant factors. It is expected that the amount will fall in a relatively broad range that can be determined through routine trials.

As used herein, the term "*function*" refers to a biological, enzymatic, or therapeutic function.

By "*functional Fgf polynucleotide*" or "*functional* FGF *polypeptide*" is meant an *Fgf* polynucleotide or an FGF polypeptide having no structural or functional defects and which do not correlate with the presence or risk of adipogenic defects including elevated or impaired adipogenesis.

The term "*gene*" as used herein refers to any and all discrete coding regions of the cell's genome, as well as associated non-coding and regulatory regions. The gene is also intended to mean the open reading frame encoding specific polypeptides, introns, and adjacent 5' and 3' non-coding nucleotide sequences involved in the regulation of expression. In this regard, the gene may further comprise control signals such as promoters, enhancers, termination and/or polyadenylation signals that are naturally associated with a given gene, or heterologous control signals. The DNA sequences may be cDNA or genomic DNA or a fragment thereof. The gene may be introduced into an appropriate vector for extrachromosomal maintenance or for integration into the host.

"*Hybridisation*" is used herein to denote the pairing of complementary nucleotide sequences to produce a DNA-DNA hybrid or a DNA-RNA hybrid. Complementary base sequences are those sequences that are related by the base-pairing rules. In DNA, A pairs with T and C pairs with G. In RNA U pairs with A and C pairs with G. In this regard, the terms "match" and "mismatch" as used herein refer to the hybridisation potential of paired nucleotides in complementary nucleic acid strands. Matched nucleotides hybridise efficiently, such as the classical A-T and G-C base pair mentioned above. Mismatches are other combinations of nucleotides that do not hybridise efficiently.

Reference herein to "*immuno-interactive*" includes reference to any interaction, reaction, or other form of association between molecules and in particular where one of the molecules is, or mimics, a component of the immune system.

By "*isolated*" is meant material that is substantially or essentially free from components that normally accompany it in its native state.

By "*modulating*" is meant increasing or decreasing, either directly or indirectly, the level or functional activity of a target molecule. For example, an agent may indirectly modulate the level/activity by interacting with a molecule other than the target molecule. In this regard, indirect modulation of a gene encoding a target polypeptide includes within its scope modulation of the expression of a first nucleic acid molecule, wherein an expression product of the first nucleic acid molecule modulates the expression of a nucleic acid molecule encoding the target polypeptide.

The term "*obesity*" as used herein includes conditions where there is an increase in body fat beyond the physical requirement as a result of excess accumulation of adipose tissue in the body. The term obesity includes but is not limited to the following conditions: adult-onset obesity; alimentary obesity; endogenous or metabolic obesity; endocrine obesity; familial obesity; hyperinsulinar obesity; hyperplastic-hypertrophic obesity; hypogonadal obesity; hypothyroid obesity; lifelong obesity; morbid obesity and exogenous obesity.

The term *"treatment of obesity"* encompasses the treatment of conditions which are secondary to obesity, which include but are not limited to cardiovascular disease, atherosclerosis, hypertension, Pickwickian syndrome and diabetes.

By "*obtained from*" is meant that a sample such as, for example, a polynucleotide extract or polypeptide extract is isolated from, or derived from, a particular source of the host. For example, the extract can be obtained from a tissue or a biological fluid isolated directly from the host.

The term "*oligonucleotide*" as used herein refers to a polymer composed of a multiplicity of nucleotide residues (deoxyribonucleotides or ribonucleotides, or related structural variants or synthetic analogues thereof) linked via phosphodiester bonds (or related structural variants or synthetic analogues thereof). Thus, while the term "oligonucleotide" typically refers to a nucleotide polymer in which the nucleotide residues and linkages between them are naturally occurring, it will be understood that the term also includes within its scope various analogues including, but not restricted to, peptide nucleic acids (PNAs), phosphoramidates, phosphorothioates, methyl phosphonates, 2-O-methyl ribonucleic acids, and the like. The exact size of the molecule can vary depending on the particular application. An oligonucleotide is typically rather short in length, generally from about 10 to 30 nucleotide residues, but the term can refer to molecules of any length, although the term "polynucleotide" or "nucleic acid" is typically used for large oligonucleotides.

By "*operably linked*" is meant that transcriptional and translational regulatory polynucleotides are positioned relative to a polypeptide-encoding polynucleotide in such a manner that the polynucleotide is transcribed and the polypeptide is translated.

The term "*patient*" refers to patients of human or other animal origin and includes any individual it is desired to examine or treat using the methods of the invention. However, it will be understood that "patient" does not imply that symptoms are present. Suitable animals that fall within the scope of the invention include, but are not restricted to, primates, livestock animals (e.g., sheep, cows, horses, donkeys, pigs), laboratory test animals (e.g., rabbits, mice, rats, guinea pigs, hamsters), companion animals (e.g., cats, dogs) and captive wild animals (e.g., foxes, deer, dingoes, avians, reptiles).

By *"pharmaceutically acceptable carrier"* is meant a solid or liquid filler, diluent or encapsulating substance that can be safely used in topical or systemic administration to a mammal.

The term *"polynucleotide"* or *"nucleic acid"* as used herein designates mRNA, RNA, cRNA, cDNA or DNA. The term typically refers to oligonucleotides greater than 30 nucleotide residues in length.

The terms *"polynucleotide variant"* and *"variant"* refer to polynucleotides displaying substantial sequence identity with a reference polynucleotide sequence or polynucleotides that hybridise with a reference sequence under stringent conditions as known in the art (see for example Sambrook et al., Molecular Cloning. A Laboratory Manual", Cold Spring Harbor Press, 1989). These terms also encompass polynucleotides in which one or more nucleotides have been added or deleted, or replaced with different nucleotides. In this regard, it is well understood in the art that certain alterations inclusive of mutations, additions, deletions and substitutions can be made to a reference polynucleotide whereby the altered polynucleotide retains a biological function or activity of the reference polynucleotide. The terms *"polynucleotide variant"* and *"variant"* also include naturally-occurring allelic variants.

*"Polypeptide", "peptide"* and *"protein"* are used interchangeably herein to refer to a polymer of amino acid residues and to variants and synthetic analogues of the same. Thus, these terms apply to amino acid polymers in which one or more amino acid residues is a synthetic non-naturally occurring amino acid, such as a chemical analogue of a corresponding naturally occurring amino acid, as well as to naturally occurring amino acid polymers.

The term *"polypeptide variant"* refers to polypeptides in which one or more amino acids have been replaced by different amino acids. It is well understood in the art that some amino acids may be changed to others with broadly similar properties without changing the nature of the activity of the polypeptide (conservative substitutions) as described hereinafter. These terms also encompass polypeptides in which one or more amino acids have been added or deleted, or replaced with different amino acids.

By *"primer"* is meant an oligonucleotide which, when paired with a strand of DNA, is capable of initiating the synthesis of a primer extension product in the presence of a suitable polymerising agent. The primer is preferably single-stranded for maximum efficiency in amplification but can alternatively be double-stranded. A primer must be sufficiently long to prime the synthesis of extension products in the presence of the polymerisation agent. The length of the primer depends on many factors, including application, temperature to be employed, template reaction conditions, other reagents, and source of primers. For example, depending on the complexity of the target sequence, the oligonucleotide primer typically contains 15 to 35 or more nucleotide residues, although it can contain fewer nucleotide residues. Primers can be large polynucleotides, such as from about 200 nucleotide residues to several kilobases or more. Primers can be selected to be "substantially complementary" to the sequence on the template to which it is designed to hybridise and serve as a site for the initiation of synthesis. By "substantially complementary", it is meant that the primer is sufficiently complementary to hybridise with a target polynucleotide. Preferably, the primer contains no mismatches with the template to which it is designed to hybridise but this is not essential. For example, non-complementary nucleotide residues can be attached to the 5' end of the primer, with the remainder of the primer sequence being complementary to the template. Alternatively, non-complementary nucleotide residues or a stretch of non-complementary nucleotide residues can be interspersed into a primer, provided that the primer sequence has sufficient complementarity with the sequence of the template to hybridise therewith and thereby form a template for synthesis of the extension product of the primer.

*"Probe"* refers to a molecule that binds to a specific sequence or sub-sequence or other moiety of another molecule. Unless otherwise indicated, the term "probe" typically refers to a polynucleotide probe that binds to another polynucleotide, often called the "target polynucleotide", through complementary base pairing. Probes can bind target polynucleotides lacking complete sequence complementarity with the probe, depending on the stringency of the hybridisation conditions. Probes can be labelled directly or indirectly.

The term *"recombinant polynucleotide*" as used herein refers to a polynucleotide formed *in vitro* by the manipulation of a polynucleotide into a form not normally found in nature. For example, the recombinant polynucleotide can be in the form of an expression vector. Generally, such expression vectors include transcriptional and translational regulatory polynucleotide operably linked to the polynucleotide.

By *"recombinant polypeptide"* is meant a polypeptide made using recombinant techniques, *i*.*e.*, through the expression of a recombinant or synthetic polynucleotide.

By *"reporter molecule"* as used in the present specification is meant a molecule that, by its chemical nature, provides an analytically identifiable signal that allows the detection of a complex comprising an antigen-binding molecule and its target antigen. The term "reporter molecule" also extends to use of cell agglutination or inhibition of agglutination such as red blood cells on latex beads, and the like.

By *"vector"* is meant a polynucleotide molecule, preferably a DNA molecule derived, for example, from a plasmid, bacteriophage, yeast or virus, into which a polynucleotide can be inserted or cloned. A vector preferably contains one or more unique restriction sites and can be capable of autonomous replication in a defined host cell including a target cell or tissue or a progenitor cell or tissue thereof, or be integrable with the genome of the defined host such that the cloned sequence is reproducible. Accordingly, the vector can be an autonomously replicating vector, *i*.*e*., a vector that exists as an extrachromosomal entity, the replication of which is independent of chromosomal replication, e.g., a linear or closed circular plasmid, an extrachromosomal element, a minichromosome, or an artificial chromosome. The vector can contain any means for assuring selfreplication. Alternatively, the vector can be one which, when introduced into the host cell, is integrated into the genome and replicated together with the chromosome(s) into which it has been integrated. A vector system can comprise a single vector or plasmid, two or more vectors or plasmids, which together contain the total DNA to be introduced into the genome of the host cell, or a transposon. The choice of the vector will typically depend on the compatibility of the vector with the host cell into which the vector is to be introduced. In the present case, the vector is preferably a viral or viral-derived vector, which is operably functional in animal and preferably mammalian cells. Such vector may be derived from a poxvirus, an adenovirus or yeast. The vector can also include a selection marker such as an antibiotic resistance gene that can be used for selection of suitable transformants. Examples of such resistance genes are known to those of skill in the art and include the *nptII* gene that confers resistance to the antibiotics kanamycin and G418 (Geneticin®) and the *hph* gene which confers resistance to the antibiotic hygromycin B.

The terms *"wild-type"* and *"normal"* are used interchangeably to refer to the phenotype that is characteristic of most of the members of the species occurring naturally and contrast for example with the phenotype of a mutant.

As used herein, underscoring or italicising the name of a gene shall indicate the gene, in contrast to its protein product, which is indicated by the name of the gene in the absence of any underscoring or italicising. For example, "*Fgf-1*" shall mean the *Fgf-1* gene, whereas "FGF-1" shall indicate the protein product or products generated from transcription and translation and alternative splicing of the *"Fgf-1"* gene.

### 2. Method of modulating adipogenesis

The present invention is predicated in part on the discovery that *in vitro* differentiation of preadipocytes into adipocytes (adipogenesis) can be enhanced by the presence of MVEC in a culture medium during the preadipocyte replication stage, and that this effect can be reproduced in the absence of MVEC by the addition of FGF-1 or FGF-2 to the culture medium. Not wishing to be bound by any one particular theory or mode of operation, the inventors consider that the *in vivo* production of FGF-1 and other members of the FGF superfamily by MVEC (or, possibly, other cell types) activate FGF receptors on adjacent preadipocytes, which directly or indirectly promotes their differentiation into adipocytes. Additionally, the present inventors have discovered that FGF-1 promotes human preadipocyte replication (more potently that IGF-1, FGF-2, or serum alone) and that FGF-1 treatment of human preadipocytes during the replication phase dramatically increases potential for subsequent differentiation (i.e., a "priming" effect). Further, the inventors have shown that this FGF-1 "priming" effect is dramatically increased by TZD treatment during differentiation, suggesting that FGF-1 is not a PPARg ligand. It has also been discovered that human preadipocytes do not produce FGF and that the pro-proliferative effect of FGF-1 is abrogated by a neutralising antibody to FGF. It is proposed, therefore, that modulators of a FGF signaling pathway, especially of the FGF-1 or FGF-2 signaling pathway, will be useful *inter alia* for the treatment or prevention of adiposity-related conditions including, but not restricted to, obesity, conditions of localised, abnormal increases in adipogenesis, cachexia and conditions of localised deficiencies in adiposity as well as in the study of excess adipogenesis and insufficient adipogenesis.

Accordingly, the present invention provides methods for modulating adipogenesis, comprising contacting a cell with an agent for a time and under conditions sufficient to modulate a FGF signaling pathway, especially a FGF-1 or FGF-2 signaling pathway. Representative members of a FGF pathway include FGFs (especially FGF-1 and FGF-2), FGFRs (e.g., FGFR-1, FGFR-2, FGFR-3, FGFR-4 and FGFR-5, especially, FGFR-1, FGFR-2, FGFR-3 and FGFR-4), HSPGs (e.g., syndecan-1, syndecan-2, syndecan-3, syndecan-4, glypican-1, glypican-2, glypican-3, glypican-4, glypican-5, glypican-6, perlecan and betaglycan), CFR, members of the SHC/FRS2-RAF/MAPKKK-MAPKK-MAPK pathway (e.g., SHC, Crk, FRS2 (FGFR substrate 2, also known as SNT-1), Src, FAK, Nck, Shb, SHP2, GRB-2, SOS, 80K-H, pp66, Gab1, P38 MAPK (ERK), PI3K, AKT, PKB, RAS, RAF, ERK1,2, MAPKKK (RAF-1), MAPKK (MEK), MARK, Jun, Fos, FPPS (farnesyl pyrophosphate synthase)), members of the PLCγ-PKC-Ca²⁺ pathway (e.g., PLCγ (phospholipase C γ), Fes, PIP2, DAG (diacyglycerol), arachidonic acid, Ca²⁺ Channel , Ca²⁺, IP3 (inositol 1,4,5 triphosphate), CaM kinase (Ca²⁺/calmodulin-dependent kinase), PKC (protein kinase C), PKA (protein kinase A), cAMP, CREB, CBP (CREB binding protein), members of the FGF-1 nuclear translocation pathway (e.g., STAT-1 and STAT-3), intracellular binding partners of FGF such as but not limited to P34 and FIF (FGF-interacting factor), and intracellular binding partners of FGFR such as STN-2, as well as their variants, including splice variants.

In accordance with the present invention, an agent can target a cell that produces a FGF (especially FGF-1 and/or FGF-2) or a cell that is the target of FGF signaling. Thus, in some embodiments, the cell is a MVEC or a MVEC precursor, whereas in others, the cell is a preadipocyte or preadipocyte precursor.

In embodiments in which a FGF-producing cell is the subject of the agent, the agent suitably modulates the expression of a *Fgf* gene (e.g., *Fgf-1, Fgf-2*) or an upstream regulator of its expression or the level or functional activity of an expression product of such genes. In these embodiments, adipogenesis is stimulated by enhancing the expression of the *Fgf* gene or the level or functional activity of its expression product or by enhancing or reducing the expression of the regulator gene or the level or functional activity of its expression product, depending upon whether it is a repressor or activator of the *Fgf* gene or its expression product. Conversely, adipogenesis is decreased or abrogated by reducing or abrogating the expression of the *Fgf* gene or the level or functional activity of its expression product or by enhancing or reducing the expression of the regulator gene or the level or functional activity of its expression product, depending upon whether it is a repressor or activator of the *Fgf* gene or its expression product, respectively.

In embodiments in which a FGF-targeted cell is the subject of the agent, the agent modulates the expression of a *Fgfr* gene (e.g., *Fgfr-1, Fgfr-2, Fgfr-3, Fgfr-4, Fgfr-5,* especially *Fgfr-1, Fgfr-3, Fgfr-4*), or a gene belonging to the same regulatory or biosynthetic pathway as the *Fgfr* gene (e.g., a gene belonging to a FGF signaling pathway, as described above), or a gene whose expression is modulated directly or indirectly by an expression product of the *Fgf* gene (e.g., PPARγ, IGFBP-3, IGFBP-6, IGF-2, IRS-2, PI3 kinase, PKCθ), or agonises or stimulates the function of a FGFR or CFR with which a FGF (e.g., FGF-1 or FGF-2) interacts. In these embodiments, adipogenesis is stimulated by enhancing the expression of the *Fgfr* gene or the level or functional activity of its expression product, or by enhancing the expression of a component of the FGF signaling pathway, or by enhancing, promoting or otherwise capacitating the interaction between a FGFR and a FGF or the interaction between a CFR and a FGF, or by stimulating dimerisation and/or phosphorylation of the FGFR. By contrast, adipogenesis is reduced or inhibited by antagonising the function of a FGFR or a CFR, including inhibiting or abrogating the interaction between a FGFR and a FGF, or between a CFR and a FGF, or by inhibiting or abrogating the interaction between an HSPG and a FGFR, by interfering with the phosphorylation of a FGFR, by interfering with components of the signaling pathway upstream or downstream of the FGF/FGFR or FGF/CFR interaction, or by interfering with the dimerisation of a FGFR.

Accordingly, when reduced adipogenesis is required, the agent is used to reduce or impair the adipogenic potential of preadipocytes including, for example, reducing or impairing the formation of adipocytes in the treatment of obesity or conditions of localised abnormal increases in adipogenesis. Conditions contemplated in such treatment regimes include pathologies which are associated with or secondary to, obesity, such as atherosclerosis, hypertension, diabetes and endocrine or other metabolic diseases or conditions. Conditions of localised, abnormal increases in adipogenesis may include adipose tumours (lipomas and liposarcomas) and lipomatosis. Alternatively, when increased adipogenesis is required, the agent is used to enhance adipogenesis including, for example, improving fat deposition in conditions associated with cachexia or in conditions of localised deficiencies in adiposity.

Suitable agents for reducing or abrogating gene expression include, but are not restricted to, oligoribonucleotide sequences, including anti-sense RNA and DNA molecules and ribozymes, that function to inhibit the translation, for example, of FGF- or FGFR-encoding mRNA. Anti-sense RNA and DNA molecules act to directly block the translation of mRNA by binding to targeted mRNA and preventing protein translation. In regard to antisense DNA, oligodeoxyribonucleotides derived from the translation initiation site, e.g., between -10 and +10 regions are preferred.

Ribozymes are enzymatic RNA molecules capable of catalysing the specific cleavage of RNA. The mechanism of ribozyme action involves sequence specific hybridisation of the ribozyme molecule to complementary target RNA, followed by a endonucleolytic cleavage. Within the scope of the invention are engineered hammerhead motif ribozyme molecules that specifically and efficiently catalyse endonucleolytic cleavage of target sequences. Specific ribozyme cleavage sites within any potential RNA target are initially identified by scanning the target molecule for ribozyme cleavage sites which include the following sequences, GUA, GUU and GUC. Once identified, short RNA sequences of between 15 and 20 ribonucleotides corresponding to the region of the target gene containing the cleavage site may be evaluated for predicted structural features such as secondary structure that may render the oligonucleotide sequence unsuitable. The suitability of candidate targets may also be evaluated by testing their accessibility to hybridisation with complementary oligonucleotides, using ribonuclease protection assays.

Both anti-sense RNA and DNA molecules and ribozymes may be prepared by any method known in the art for the synthesis of RNA molecules. These include techniques for chemically synthesising oligodeoxyribonucleotides well known in the art such as for example solid phase phosphoramidite chemical synthesis. Alternatively, RNA molecules may be generated by *in vitro* and *in vivo* transcription of DNA sequences encoding the antisense RNA molecule. Such DNA sequences may be incorporated into a wide variety of vectors which incorporate suitable RNA polymerase promoters such as the T7 or SP6 polymerase promoters. Alternatively, antisense cDNA constructs that synthesise antisense RNA constitutively or inducibly, depending on the promoter used, can be introduced stably into cell lines.

Various modifications to the DNA molecules may be introduced as a means of increasing intracellular stability and half-life. Possible modifications include but are not limited to the addition of flanking sequences of ribo- or deoxy- nucleotides to the 5' or 3' ends of the molecule or the use of phosphorothioate or 2' O-methyl rather than phosphodiesterase linkages within the oligodeoxyribonucleotide backbone.

Alternatively, RNA molecules that mediate RNA interference (RNAi) of a target gene or gene transcript can be used to reduce or abrogate gene expression. RNAi refers to interference with or destruction of the product of a target gene by introducing a single stranded, and typically a double stranded RNA (dsRNA) that is homologous to the transcript of a target gene. Thus, in some embodiments, dsRNA *per se* and especially dsRNA-producing constructs corresponding to at least a portion of a target gene may be used to reduce or abrogate its expression. RNAi-mediated inhibition of gene expression may be accomplished using any of the techniques reported in the art, for instance by transfecting a nucleic acid construct encoding a stem-loop or hairpin RNA structure into the genome of the target cell, or by expressing a transfected nucleic acid construct having homology for a target gene from between convergent promoters, or as a head to head or tail to tail duplication from behind a single promoter. Any similar construct may be used so long as it produces a single RNA having the ability to fold back on itself and produce a dsRNA, or so long as it produces two separate RNA transcripts which then anneal to form a dsRNA having homology to a target gene.

Absolute homology is not required for RNAi, with a lower threshold being described at about 85% homology for a dsRNA of about 200 base pairs (Plasterk and Ketting, 2000, Current Opinion in Genetics and Dev. 10: 562-67). Therefore, depending on the length of the dsRNA, the RNAi-encoding nucleic acids can vary in the level of homology they contain toward the target gene transcript, *i*.*e*., with dsRNAs of 100 to 200 base pairs having at least about 85% homology with the target gene, and longer dsRNAs, *i.e.*, 300 to 100 base pairs, having at least about 75% homology to the target gene. RNA-encoding constructs that express a single RNA transcript designed to anneal to a separately expressed RNA, or single constructs expressing separate transcripts from convergent promoters, are preferably at least about 100 nucleotides in length. RNA-encoding constructs that express a single RNA designed to form a dsRNA *via* internal folding are preferably at least about 200 nucleotides in length.

The promoter used to express the dsRNA-forming construct may be any type of promoter if the resulting dsRNA is specific for a gene product in the cell lineage targeted for destruction. Alternatively, the promoter may be lineage specific in that it is only expressed in cells of a particular development lineage. This might be advantageous where some overlap in homology is observed with a gene that is expressed in a non-targeted cell lineage. The promoter may also be inducible by externally controlled factors, or by intracellular environmental factors.

In other embodiments, RNA molecules of about 21 to about 23 nucleotides, which direct cleavage of specific mRNA to which they correspond, as for example described by Tuschl *et al.* in U.S. Patent Application No. 20020086356, can be utilised for mediating RNAi. Such 21-23 nt RNA molecules can comprise a 3' hydroxyl group, can be single-stranded or double stranded (as two 21-23 nt RNAs) wherein the dsRNA molecules can be blunt ended or comprise overhanging ends (e.g., 5', 3').

In accordance with the present invention, various stages of a FGF signaling pathway can be targeted for modulating adipogenesis. In some embodiments, the level or concentration of a FGF is the subject of the targeting. Suitably, the level or functional activity of a FGF, especially of an extracellular FGF, is reduced through use of anti-FGF antigen-binding molecules (e.g., neutralising antibodies) as sold commercially for example by R & D systems AF232 (R&D Systems Inc. Minneapolis, MN) or as disclosed in Cancer Res 1988. 48:4266.

In other embodiments, the FGF-FGFR binding or activation is the subject of the targeting. For example, stimulation of FGFR signaling can be achieved by overexpression of the FGFR, or through mutations that promote FGFR dimerisation/oligomerisation in the absence of ligand and subsequent constitutive activation. Alternatively, non-ligand molecules that induce receptor dimerisation can be used to produce a similar effect. Receptor mutations can also induce dissociation of biological effects, and could be utilised to "tailor" FGF-responses.

In other embodiments, inhibition or abrogation of FGFR signaling is achieved through reduction in FGFR expression, FGFR mutation (in particular, but not exclusively, of phosphorylation sites), prevention of receptor aggregation or through approaches that interfere with ligand-receptor interaction *via* blockade of the active binding sites or relevant associated motifs. Such strategies include blocking antibodies to the receptors and small molecule inhibitors of binding. Pharmacological strategies to impair receptor phosphorylation can also be effective. Exemplary FGFR antagonists include soluble forms of FGFR including, but not restricted to, soluble recombinant FGFR-1(IIIc)/Fc chimeras, soluble recombinant FGFR-2/Fc chimeras and soluble recombinant FGFR-3/Fc chimeras, as disclosed for example in Oncogene 1991, 6:1195 and in FASEB J 1992. 6:3362. The present invention also contemplates the use of FGPR antagonistic antigen-binding molecules with varying blocking capacities, as disclosed for example in Cancer Res 1988. 48:4266. In other embodiments, metal chelators (e.g., EDTA or EGTA) can be used to block FGFR dimerisation, as disclosed for example in J Biol Chem 1992. 267:11307 and FGFR-binding peptides can be used to antagonise the activity or activation of a FGFR (e.g., the FGFR⁷³⁰(p)Y disclosed in Cell Growth and Diff. 2001, and the synthetic peptide Ac-ValTyrMetSerProPhe-NH₂ disclosed in IUBMB Life 2002. 54:67. The present invention also contemplates the use of FGF-2 inhibitors such as TMPP (Cardiovascular Res 2002. 53:232), FGFR1 tyrosine kinase inhibitors such as PD161570 (Life Sciences 1998. 62:143).

In other embodiments, the subject of the targeting is an HSPG. It is known in this regard that modification of HPSG expression or type effects FGF signaling and that HPSG mutations (natural or artificial) are associated with modulation of FGF signaling. For example, mutations in Glypican-3 as seen in the Simpson-Golabi-Behmel syndrome are associated with upregulated FGF-1 signalling. HPSG expression can be reduced pharmacologically in a number of non-specific and specific ways, leading to alteration in FGF signaling. Such strategies have been developed in an effort to reduce angiogenesis and tumour development. Other molecules that function in a manner akin to the HSPGs (i.e., that regulate the ligand-receptor complex or it's activity) can also modulate FGF signaling. Exemplary HSPG antagonists include, but are not limited to, sucrose octasulfate (Mol Cell Biol 2002. 22:7184), suramins (J Mol Biol 1998. 281:899), suradistas (J Mol Biol 1998. 281:899), TNP-470 (PNAS 2002. 99:10730), angiostatin (PNAS 2002. 99:10730), endostatin (PNAS 2001. 98:12509 and Human Gene Therapy. 2001. 12:347), heparanase inhibitors such as phosphomannopentaose sulfate (PI-88) (Cancer Research 1999. 59:3433), maltohexaose sulfate (Cancer Research 1999. 59:3433), heparinases (J. Biol Chem 1997. 272:12415 and J. Biol. Chem 1994. 269:32279), heparatinases (J. Biol. Chem 1994. 269:32279) and sodium chlorate (J. Biol. Chem 1994. 269:32279).

In still other embodiments, the subject of the targeting is a component of post-receptor FGF signal transduction. Regulation of post-FGFR signaling can increase or decrease specific biological effects of the FGFs. Such strategies also have the potential for cell- or tissue-specific effects to be obtained. Whilst, as outlined above, the signal transduction pathways utilised by the FGFRs are often not unique to the ligand or receptor, regulation of FGF signaling through modulation of the signaling pathways has been well demonstrated.. Representative antagonists of the SHC/FRS2-RAF/MAPKKK-MAPKK-MAPK pathway include, but are not restricted to, PKC inhibitors such as calphostin C (Cal C) (J Biol Chem. 1999 274:18243), MEK inhibitors such as PD 98059 (PD) (Diabetes 2003. 52:43 and JBC 1998. 273:32111), PI3-K inhibitors such as Ly 294002 (LY) (Cellular Signalling 2001. 13:363 and J. Neurochem. 2002. 81:365), control compounds for SB 190 such as SB 202474 (SB 474) (JBC 1998, 273:32111), SB203580 (Diabetes 2003. 52:43 and JBC 1998. 273:32111), SB 202190 (JBC 1998. 273:32111), 12-*O*-tetradecanoyl phorbol 13-acetate (TPA) (Oncogene 2002. 21:1978) and PD 98059. Alternatively, the PLCγ-PI3K-PKC-Ca²⁺ pathway can be targeted and in this regard, there are numerous studies supporting the hypothesis that inhibition of this pathway at any level can interfere with growth factor signaling, including that of FGFs. Exemplary inhibitors contemplated for use in the practice of the invention include, but are not limited to, phospholipase C inhibitors such as U-73122 (Calbiochem).

In still other embodiments, the subject of the targeting is the CFR. In these embodiments, overexpression of CFR will lead to decreased intracellular accumulation of FGF-1 and FGF-2. Such strategies could regulate FGF actions, in particular by regulating presumed direct transcriptional effects.

The present invention also contemplates the use in the above method of gene or expression product inhibitors identified according to methods described for example in Section 3, *infra.*

Agents that may be used to enhance the activity of target polypeptide include any suitable inducer or stabilising/activating agents which can be identified or produced by standard protocols as disclosed for example in Section 3 *infra* or using non-human animal models. In this instance, the agent may comprise at least a biologically active fragment of the target polypeptide or polynucleotide encoding the full-length target polypeptide or biologically active fragment thereof. Exemplary agents of this type include a FGFR or FGF agonising antigen-binding molecule, a *Fgf* polynucleotide or a FGF polypeptide or a polynucleotide whose expression product enhances, promotes or otherwise capacitates the interaction between a FGF and a FGFR, or the polypeptide expression product of the polynucleotide. Sequence information for producing *Fgf* polynucleotides and FGF polypeptides is available in publicly available databases such as GenBank and EMBL. Such molecules can be easily manufactured by persons of skill in the art using standard techniques.

The modulatory agents of the invention will suitably affect or modulate adipogenesis. Accordingly, the cells that are the subject of testing are preferably MVEC or progenitors thereof, which are a source of FGFs, or preadipocytes that may express FGF receptors which are activated by FGFs. Preadipocytes are the cell type whose differentiation *via* adipogenesis creates new adipocytes. The accumulation of the latter cell type leads to increases in adiposity which precede obesity, and conversely, excessive loss of adipocytes in the absence of adipogenesis leads to excessively low adiposity, as occurs in cachexia or conditions of localised deficiencies in adiposity. Suitable assays for testing the effects of modulatory agents on MVEC include, but are not restricted to, their co-culture with preadipocytes in the presence of putative FGF modulatory agents or FGFR modulatory agents. The ability of modulatory agents to inhibit or stimulate the differentiation potential of preadipocytes can be measured using cultured preadipocytes or *in vivo* by administering molecules of the present invention to the appropriate animal model. The inventors of the present invention have established a system for obtaining biopsies of omental and subcutaneous adipose tissue from individuals undergoing elective abdominal surgery and using the preadipocytes and MVEC from such biopsy material for cell culture. Assays for measuring proliferation and differentiation potential are well known in the art. Subcutaneous and omental preadipocytes are plated then exposed to MVEC-conditioned growth medium in the presence or absence of putative FGF or FGFR modulatory agents. Assays for measuring preadipocyte proliferation and differentiation are also well known in the art. For example, assays measuring proliferation include such assays as assessment of preadipocyte cell number following exposure to a proliferative growth medium using a formazan colorimetric assay (Promega). Preadipocyte differentiation potential is assessed by the measurement of glycerol-3-phosphate dehydrogenase (G3PDH) enzyme activity and triacylglycerol accumulation.

*In vivo* evaluation tools, which are well known to practitioners in the art, are available for evaluating the effect of FGF signalling pathway-modulatory agents as described herein on the differentiation potential of preadipocytes into adipocytes. Such differentiation results in the accumulation of adipose tissue, and assay means for measuring the amount of such tissue in a patient include skin fold measurements using an adipometer. This assay involves the integration of skin fold thicknesses from suitable areas (e.g., triceps, biceps, subscapular and suprailiac regions) to obtain a body fat percentage value. Other *in vivo* assays include underwater weighing, bioelectrical impedance, dual energy x-ray absorptiometry and radiological imaging (e.g., computerised tomography or magnetic resonance imaging).

FGF signalling pathway-modulatory agents as described herein may also have applications for enhancing adipogenesis in conditions where severe depletion of fat deposits occur, generally referred to herein by the terms cachexia and cachexia-related conditions. Other applications include in the clinical management of conditions where localised deficiencies in adipogenesis exist. Such conditions include lipodystrophy and regional loss of adipose tissue from physical injury, burns or atrophic disease. Such conditions may result from *inter alia* cancer, cardiac disease, malaria and advanced renal failure. The methods of the present invention may prevent or retard adipose tissue wastage associated with such pathological conditions.

### 3. Identification of target molecule modulators

The invention also features methods of screening for an agent that modulates a FGF signaling pathway, including modulating the expression of a gene or the level and/ or functional activity of an expression product of that gene, wherein the gene is selected from a *Fgf* gene, a *Fgfr* gene, a gene relating to the same regulatory or biosynthetic pathway as the *Fgf* gene or a *Fgfr* gene, a gene relating to the same regulatory or biosynthetic pathway as the FGFR gene, or a gene whose expression product modulates (e.g., promotes, enhances or capacitates; or inhibits or impairs) the interaction between a FGF and a FGFR, or a gene whose expression is modulated directly or indirectly by an expression product of the *Fgf* gene, or that agonises or antagonises the function of a FGFR with which a FGF interacts.

In some embodiments, the methods comprise: (1) contacting a preparation with a test agent, wherein the preparation contains (i) a polypeptide comprising an amino acid sequence corresponding to at least a biologically active fragment of a polypeptide component of the FGF signaling pathway, or to a variant or derivative thereof; or (ii) a polynucleotide comprising at least a portion of a genetic sequence that regulates the component, which is operably linked to a reporter gene; and (2) detecting a change in the level and/or functional activity of the polypeptide component, or an expression product of the reporter gene, relative to a normal or reference level and/or functional activity in the absence of the test agent, which indicates that the agent modulates the FGF signaling pathway.

Any suitable assay for detecting, measuring or otherwise determining modulation of adipogenesis (e.g., such as by detecting preadipocyte proliferation and differentiation potential), is contemplated by the present invention. Assays of a suitable nature are known to persons of skill in the art and examples of these are described in Section 2 *supra*

Modulators falling within the scope of the present invention include agonists and antagonists of a FGF signaling pathway including antagonistic antigen-binding molecules, and inhibitor peptide fragments, antisense molecules, ribozymes, RNAi molecules and co-suppression molecules, phospholipase C inhibitors and kinase inhibitors, as for example described in Section 2. Agonists include agonistic antigen-binding molecules, components of the FGF signaling pathway or their biologically active fragments, variants and derivatives, molecules which increase promoter activity or interfere with negative regulatory mechanisms and molecules which overcome any negative regulatory mechanism.

Candidate agents encompass numerous chemical classes, though typically they are organic molecules, preferably small organic compounds having a molecular weight of more than 50 and less than about 2,500 Dalton. Candidate agents comprise functional groups necessary for structural interaction with proteins, particularly hydrogen bonding, and typically include at least an amine, carbonyl, hydroxyl or carboxyl group, preferably at least two of the functional chemical groups. The candidate agent often comprises cyclical carbon or heterocyclic structures or aromatic or polyaromatic structures substituted with one or more of the above functional groups. Candidate agents are also found among biomolecules including, but not limited to: peptides, saccharides, fatty acids, steroids, purines, pyrimidines, derivatives, structural analogues or combinations thereof.

Small (non-peptide) molecule modulators of a FGF polypeptide or a FGFR polypeptide, are particularly preferred. In this regard, small molecules are particularly preferred because such molecules are more readily absorbed after oral administration, have fewer potential antigenic determinants, or are more likely to cross the cell membrane than larger, protein-based pharmaceuticals. Small organic molecules may also have the ability to gain entry into an appropriate cell and affect the expression of a gene (eg by interacting with the regulatory region or transcription factors involved in gene expression); or affect the activity of a gene by inhibiting or enhancing the binding of accessory molecules.

Alternatively, libraries of natural compounds in the form of bacterial, fungal, plant and animal extracts are available or readily produced. Additionally, natural or synthetically produced libraries and compounds are readily modified through conventional chemical, physical and biochemical means, and may be used to produce combinatorial libraries. Known pharmacological agents may be subjected to directed or random chemical modifications, such as acylation, alkylation, esterification, amidification, etc to produce structural analogues.

Screening may also be directed to known pharmacologically active compounds and chemical analogues thereof.

Screening for modulatory agents according to the invention can be achieved by any suitable method. For example, the method may include contacting a cell expressing a polynucleotide corresponding to a *Fgf* gene or a *Fgfr* gene or to a gene belonging to the same regulatory or biosynthetic pathway as a *Fgf* or *Fgfr* gene, with an agent suspected of having the modulatory activity and screening for the modulation of the level or functional activity of a protein encoded by the polynucleotide, or the modulation of the level of a transcript encoded by the polynuclcotide, or the modulation of the activity or expression of a downstream cellular target of the protein or of the transcript (hereafter referred to as target molecules). Detecting such modulation can be achieved utilising techniques including, but not restricted to, ELISA, cell-based ELISA, inhibition ELISA, Western blots, immunoprecipitation, slot or dot blot assays, immunostaining, RIA, scintillation proximity assays, fluorescent immunoassays using antigen-binding molecule conjugates or antigen conjugates of fluorescent substances such as fluorescein or rhodamine, Ouchterlony double diffusion analysis, immunoassays employing an avidin-biotin or a streptavidin-biotin detection system, and nucleic acid detection assays including reverse transcriptase polymerase chain reaction (RT-PCR).

It will be understood that a polynucleotide from which a target molecule of interest is regulated or expressed may be naturally occurring in the cell which is the subject of testing or it may have been introduced into the host cell for the purpose of testing. Further, the naturally-occurring or introduced polynucleotide may be constitutively expressed - thereby providing a model useful in screening for agents which down-regulate expression of an encoded product of the sequence wherein the down regulation can be at the nucleic acid or expression product level - or may require activation - thereby providing a model useful in screening for agents that up-regulate expression of an encoded product of the sequence. Further, to the extent that a polynucleotide is introduced into a cell, that polynucleotide may comprise the entire coding sequence which codes for a target protein or it may comprise a portion of that coding sequence (e.g., the FGF binding domain of a FGFR, or the FGFR binding domain of a *Fgf*, or the HSPG-binding domain of a FGFR) or a portion that regulates expression of a product encoded by the polynucleotide (e.g., a promoter). For example, the promoter that is naturally associated with the polynucleotide may be introduced into the cell that is the subject of testing. In this regard, where only the promoter is utilised, detecting modulation of the promoter activity can be achieved, for example, by operably linking the promoter to a suitable reporter polynucleotide including, but not restricted to, green fluorescent protein (GFP), luciferase, β-galactosidase and catecholamine acetyl transferase (CAT). Modulation of expression may be determined by measuring the activity associated with the reporter polynucleotide.

In another example, the subject of detection could be a downstream regulatory target of the target molecule, rather than the target molecule itself or the reporter molecule operably linked to a promoter of a gene encoding a product the expression of which is regulated by the target protein.

These methods provide a mechanism for performing high throughput screening of putative modulatory agents such as proteinaceous or non-proteinaceous agents comprising synthetic, combinatorial, chemical and natural libraries. These methods will also facilitate the detection of agents which bind either the polynucleotide encoding the target molecule or which modulate the expression of an upstream molecule, which subsequently modulates the expression of the polynucleotide encoding the target molecule. Accordingly, these methods provide a mechanism of detecting agents that either directly or indirectly modulate the expression or activity of a target molecule according to the invention.

In a series of embodiments, the present invention provides assays for identifying small molecules or other compounds (*ie* modulatory agents) which are capable of inducing or inhibiting the level and/or functional activity of target molecules according to the invention. The assays may be performed *in vitro* using non-transformed cells, immortalised cell lines, or recombinant cell lines. In addition, the assays may detect the presence of increased or decreased expression of genes or production of proteins on the basis of increased or decreased mRNA expression (using, for example, the nucleic acid probes disclosed herein), increased or decreased levels of protein products (using, for example, the antigen binding molecules disclosed herein), or increased or decreased levels of expression of a reporter gene (e.g., GFP, β-galactosidase or luciferase) operably linked to a target molecule-related gene regulatory region in a recombinant construct.

Thus, for example, one may culture cells which produce a particular target molecule and add to the culture medium one or more test compounds. After allowing a sufficient period of time (e.g., 6-72 hours) for the compound to induce or inhibit the level or functional activity of the target molecule, any change in the level from an established baseline may be detected using any of the techniques described above and well known in the art. In particularly preferred embodiments, the cells are preadipocytes or microvascular endothelial cells (MVEC). Using suitable nucleic acid probes or antigen-binding molecules, detection of changes in the level and or functional activity of a target molecule, and thus identification of the compound as agonist or antagonist of the target molecule, requires only routine experimentation.

In some embodiments, recombinant assays are employed in which a reporter gene encoding, for example, GFP, β-galactosidase or luciferase is operably linked to the 5' regulatory regions of a target molecule related gene. Such regulatory regions may be easily isolated and cloned by one of ordinary skill in the art. The reporter gene and regulatory regions are joined inframe (or in each of the three possible reading frames) so that transcription and translation of the reporter gene may proceed under the control of the regulatory elements of the target molecule related gene. The recombinant construct may then be introduced into any appropriate cell type although mammalian cells are preferred, and human cells are most preferred. The transformed cells may be grown in culture and, after establishing the baseline level of expression of the reporter gene, test compounds may be added to the medium. The ease of detection of the expression of the reporter gene provides for a rapid, high throughput assay for the identification of agonists or antagonists of the target molecules of the invention.

Compounds identified by this method will have potential utility in modifying the expression of target molecule related genes *in vivo.* These compounds may be further tested in the animal models to identify those compounds having the most potent *in vivo* effects. In addition, as described above with respect to small molecules having target polypeptide binding activity, these molecules may serve as "lead compounds" for the further development of pharmaceuticals by, for example, subjecting the compounds to sequential modifications, molecular modelling, and other routine procedures employed in rational drug design.

In other embodiments, methods of identifying agents that inhibit FGF activity are provided in which a purified preparation of a FGF protein is incubated in the presence and absence of a candidate agent under conditions in which the FGF is active, and the level of FGF activity is measured by a suitable assay. For example, a FGF inhibitor can be identified by measuring the ability of a candidate agent to decrease FGF activity in a cell (e.g., a MVEC and a preadipocyte). In one embodiment of this method, a MVEC that is capable of expressing a *Fgf*, is co-cultured with preadipocytes, and the cells in the culture medium are exposed to, or cultured in the presence and absence of, the candidate agent under conditions in which the FGF is active in the cells, and an activity relating to adipogenesis such as the enhancement of the differentiation potential of preadipocytes is detected. An agent tests positive if it inhibits this activity.

In still other embodiments, a method of identifying agents that increase FGF activity is provided in which a purified preparation of a FGF protein is incubated in the presence and absence of a candidate agent under conditions in which the FGF is active, and the level of FGF activity is measured by a suitable assay. For example, a FGF stimulator or activator can be identified by measuring the ability of a candidate agent to increase FGF activity or activation in a cell (e.g., a MVEC and a preadipocyte). In one embodiment of this method, a MVEC that is capable of expressing a *Fgf*, is co-cultured with preadipocytes, and the cells in the culture medium are exposed to, or cultured in the presence and absence of, the candidate agent under conditions in which the FGF is active in the cells, and an activity relating to adipogenesis such as enhancing the differentiation potential of preadipocytes is detected. An agent tests positive if it elevates this activity.

In still other embodiments, methods of identifying agents that inhibit or prevent FGFR activation are provided in which a purified preparation of a FGFR protein is incubated in the presence and absence of a candidate agent under conditions in which the FGFR is able to bind a FGF ligand, and the level of FGFR activation is measured by a suitable assay. For example, a FGFR antagonist can be identified by measuring the ability of a candidate agent to decrease FGFR activation in a cell (e.g. a preadipocyte) from a baseline value in the presence of receptor ligand. In one embodiment of this method, a preadipocyte that is capable of expressing a *Fgfr*, is co-cultured with MVEC, and the cells in the culture medium are exposed to, or cultured in the presence and absence of, the candidate agent under conditions in which the FGF is active in the cells, and an activity relating to adipogenesis such as enhancement of the differentiation potential of preadipocytes is detected. An agent tests positive if it inhibits this activity.

In other embodiments, methods of identifying agents that enhance FGFR activation are provided in which a purified preparation of a FGFR protein is incubated in the presence and absence of a candidate agent under conditions in which the FGFR is able to bind a FGF ligand, and the level of FGFR activation is measured by a suitable assay. For example, a FGFR agonist can be identified by measuring the ability of a candidate agent to enhance basal FGFR activation in a cell (e.g., a preadipocyte) from a baseline value in the presence of receptor ligand. In one embodiment of this method, a preadipocyte that is capable of expressing a *Fgfr*, is co-cultured with MVEC, and the cells in the culture medium are exposed to, or cultured in the presence and absence of, the candidate agent under conditions in which the FGF is active in the cells, and an activity relating to adipogenesis such as enhancement of the differentiation potential of preadipocytes is detected. An agent tests positive if enhances or promotes this activity.

In still other embodiments, random peptide libraries consisting of all possible combinations of amino acids attached to a solid phase support may be used to identify peptides that are able to bind to a target molecule or to a functional domain thereof. Identification of molecules that are able to bind to a target molecule may be accomplished by screening a peptide library with a recombinant soluble target molecule. The target molecule may be purified, recombinantly expressed or synthesised by any suitable technique. Such molecules may be conveniently prepared by a person skilled in the art using standard protocols as for example described in Sambrook, *et al.,* (1989, supra) in particular Sections 16 and 17; Ausubel et al., ("Current Protocols in Molecular Biology", John Wiley & Sons Inc, 1994-1998), in particular Chapters 10 and 16; and Coligan et al., ("Current Protocols in Immunology", (John Wiley & Sons, Inc, 1995-1997), in particular Chapters 1, 5 and 6. Alternatively, a target polypeptide according to the invention may be synthesised using solution synthesis or solid phase synthesis as described, for example, in Chapter 9 of Atherton and Shephard (*supra*) and in Roberge et al (1995, Science 269: 202).

To identify and isolate the peptide/solid phase support that interacts and forms a complex with a target molecule, suitably a target polypeptide, it may be necessary to label or "tag" the target polypeptide. The target polypeptide may be conjugated to any suitable reporter molecule, including enzymes such as alkaline phosphatase and horseradish peroxidase and fluorescent reporter molecules such as fluorescein isothyiocynate (FITC), phycoerythrin (PE) and rhodamine. Conjugation of any given reporter molecule, with target polypeptide, may be performed using techniques that are routine in the art. Alternatively, target polypeptide expression vectors may be engineered to express a chimeric target polypeptide containing an epitope for which a commercially available antigen-binding molecule exists. The epitope specific antigen-binding molecule may be tagged using methods well known in the art including labelling with enzymes, fluorescent dyes or coloured or magnetic beads.

For example, the "tagged" target polypeptide conjugate is incubated with the random peptide library for 30 minutes to one hour at 22° C to allow complex formation between target polypeptide and peptide species within the library. The library is then washed to remove any unbound target polypeptide. If the target polypeptide has been conjugated to alkaline phosphatase or horseradish peroxidase the whole library is poured into a petri dish containing a substrate for either alkaline phosphatase or peroxidase, for example, 5-bromo-4-chloro-3-indoyl phosphate (BCIP) or 3,3',4,4"-diamnobenzidine (DAB), respectively. After incubating for several minutes, the peptide/solid phase-target polypeptide complex changes colour, and can be easily identified and isolated physically under a dissecting microscope with a micromanipulator. If a fluorescently tagged target polypeptide has been used, complexes may be isolated by fluorescent activated sorting. If a chimeric target polypeptide having a heterologous epitope has been used, detection of the peptide/target polypeptide complex may be accomplished by using a labelled epitope specific antigen-binding molecule. Once isolated, the identity of the peptide attached to the solid phase support may be determined by peptide sequencing.

### 4. Methods of detecting expression of genes involved in an FGF signaling pathway

Since genes of the FGF signaling pathway (e.g., *Fgf* genes and *Fgfr* genes) are considered to be associated with adipogenesis, and in particular, in priming preadipocytes for differentiation, it is proposed that aberrations in expression of such genes may underlie or contribute to dysfunctional adipogenesis including elevated adipogenesis that may be linked with a predisposition to developing obesity or obesity-related conditions, including but not limited to: familial obesity, atherosclerosis, hypertension and diabetes. Accordingly, the present invention contemplates a method for detecting the presence or diagnosing the risk of obesity in a patient, comprising determining the presence of an aberrant gene involved in a FGF signaling pathway (e.g., an aberrant *Fgf* gene or *Fgfr* gene) or an aberrant expression product of that gene in a biological sample obtained from the patient, wherein the aberrant gene or the aberrant expression product correlates with the presence of or predisposition to developing obesity or obesity-related conditions.

In some embodiments, the method comprises detecting a level and/or functional activity of an expression product of the gene, which is different than a normal reference level and/or functional activity of that expression product. For example, the presence of, or the probable affliction with, obesity is diagnosed when a *Fgf gene* product or a *Fgfr* gene product is expressed at a detectably higher level compared to the level at which it is expressed in normal, non-obese patients or in non-affected patients. Alternatively, obesity is diagnosed by detecting a level or functional activity of an expression product of a *Fgf* gene or a *Fgfr* gene, which is increased or elevated relative to a normal, non-obese reference level or functional activity of that gene.

Thus, it will be desirable to qualitatively or quantitatively determine protein levels or transcription levels of components of a FGF signaling pathway. Alternatively or additionally, it may be desirable to search for aberrant structural genes of the FGF signaling pathway and their regulatory regions.

The biological sample can be any suitable tissue (e.g., a biopsy of subcutaneous connective tissue or omental tissue) or fluid.

### 4.1 Genetic Diagnosis

One embodiment of the instant invention comprises a method for detecting an increase in the expression of a gene involved in a FGF signaling pathway. For example, one may detect the expression of a *Fgf* gene or a *Fgfr* gene by qualitatively or quantitatively determining the transcripts of the *Fgf* gene in a cell (e.g., a MVEC) or the transcripts of a *Fgfr* gene in a cell (e.g., a preadipocyte). Another embodiment of the instant invention comprises a method for detecting an increase in the expression or function of a gene involved in a FGF signaling pathway (e.g., a *Fgf* gene or a *Fgfr* gene) by examining the genes and transcripts of a cell (e.g., a MVEC). In these embodiments, nucleic acid can be isolated from cells contained in the biological sample, according to standard methodologies (Sambrook, et al., "Molecular Cloning. A Laboratory Manual", Cold Spring Harbor Press, 1989; Ausubel et al., "Current Protocols in Molecular Biology", John Wiley & Sons Inc, 1994-1998). The nucleic acid may be genomic DNA or fractionated or whole cell RNA. Where RNA is used, it may be desired to convert the RNA to a complementary DNA. In one embodiment, the RNA is whole cell RNA; in another, it is poly-A RNA. In one embodiment, the nucleic acid is amplified by a nucleic acid amplification technique. Suitable nucleic acid amplification techniques are well known to the skilled person, and include the polymerase chain reaction (PCR) as for example described in Ausubel *et al*. (*supra*); strand displacement amplification (SDA) as for example described in U.S. Patent No 5,422,252; rolling circle replication (RCR) as for example described in Liu et al., (1996) and International application WO 92/01813) and Lizardi et al., (International Application WO 97/19193); nucleic acid sequencebased amplification (NASBA) as for example described by Sooknanan et al., (1994, Biotechniques 17:1077-1080); and Q-β replicase amplification as for example described by Tyagi et al., (1996, Proc. Natl. Acad. Sci. USA 93: 5395-5400).

Depending on the format, the specific nucleic acid of interest is identified in the sample directly using amplification or with a second, known nucleic acid following amplification. Next, the identified product is detected. In certain applications, the detection may be performed by visual means (e.g., ethidium bromide staining of a gel). Alternatively, the detection may involve indirect identification of the product via chemiluminescence, radioactive scintigraphy of radiolabel or fluorescent label or even via a system using electrical or thermal impulse signals (Affymax Technology; Bellus, 1994, J Macromol. Sci. Pure, Appl. Chem., A31(1): 1355-1376).

Following detection, one may compare the results seen in a given patient with a control reaction or a statistically significant reference group of normal subjects, In this way, it is possible to correlate the amount of an expression product detected with the progression or severity of the obesity.

In addition to determining levels of transcripts, it also may prove useful to examine various types of defects. These defects could include deletions, insertions, point mutations and duplications. Point mutations result in stop codons, frameshift mutations or amino acid substitutions. Somatic mutations are those occurring in non-germline tissues. Germ-line tissue can occur in any tissue and are inherited. Mutations in and outside the coding region also may affect the amount of FGF signaling pathway component produced, both by altering the transcription of the gene or in stabilising or otherwise altering the processing of either the transcript (mRNA) or protein.

A variety of different assays are contemplated in this regard, including but not limited to, fluorescent *in situ* hybridisation (FISH), direct DNA sequencing, pulse field gel electrophoresis (PFGE) analysis, Southern or Northern blotting, single-stranded conformation analysis (SSCA), RNasc protection assay, allele-specific oligonucleotide (ASO), dot blot analysis, denaturing gradient gel electrophoresis, RFLP and PCR-SSCP. Also contemplated by the present invention are chip-based DNA technologies such as those described by Hacia et al. (1996, Nature Genetics 14: 441-447) and Shoemaker et al. (1996, Nature Genetics 14: 450-456). Briefly, these techniques involve quantitative methods for analysing large numbers of genes rapidly and accurately. By tagging genes with oligonucleotides or using fixed probe arrays, one can employ chip technology to segregate target molecules as high density arrays and screen these molecules on the basis of hybridisation. See also Pease et al. (1994, Proc, Natl. Acad. Sci. U.S.A. 91: 5022-5026); Fodor et al. (1991, Science 251: 767-773).

### 4.2 Protein-based diagnostics

### 4.2.1 Antigen-binding molecules

Antigen-binding molecules that are immuno-interactive with a target molecule of the present invention can be used in measuring an increase or decrease in the expression of FGF signaling pathway genes. Thus, the present invention also contemplates antigen-binding molecules that bind specifically to an expression product of a gene involved in a FGF signaling pathway (e.g., FGF or a FGFR polypeptide or proteins that regulate or otherwise influence the level and/or functional activity of one or more FGF polypeptides or FGFR polypeptides). For example, the antigen-binding molecules may comprise whole polyclonal antibodies. Such antibodies may be prepared, for example, by injecting a target molecule of the invention into a production species, which may include mice or rabbits, to obtain polyclonal antisera. Methods of producing polyclonal antibodies are well known to those skilled in the art. Exemplary protocols which may be used are described for example in Coligan et al., "Current Protocols In Immunology", (John Wiley & Sons, Inc, 1991), and Ausubel *et al*., (1994-1998, *supra*), in particular Section III of Chapter 11.

In lieu of the polyclonal antisera obtained in the production species, monoclonal antibodies may be produced using the standard method as described, for example, by Köhler and Milstein (1975, Nature 256, 495-497), or by more recent modifications thereof as described, for example, in Coligan *et al*., (1991, *supra*) by immortalising spleen or other antibody-producing cells derived from a production species which has been inoculated with target molecule of the invention.

The invention also contemplates as antigen-binding molecules Fv, Fab, Fab' and F(ab')₂ immunoglobulin fragments. Alternatively, the antigen-binding molecule may be in the form of a synthetic stabilised Fv fragment, a single variable region domain (also known as a dAbs), a "minibody" and the like as known in the art.

Also contemplated as antigen binding molecules are humanised antibodies. Humanised antibodies are produced by transferring complementary determining regions from heavy and light variable chains of a non human (e.g., rodent, preferably mouse) immunoglobulin into a human variable domain. Typical residues of human antibodies are then substituted in the framework regions of the non human counterparts. The use of antibody components derived from humanised antibodies obviates potential problems associated with the immunogenicity of non human constant regions. General techniques for cloning non human, particular murine, immunoglobulin variable domains are described, for example, by Orlandi et al. (1989, Proc. Natl. Acad. Sci. USA 86: 3833). Techniques for producing humanised monoclonal antibodies are described, for example, by Jones et al. (1986, Nature 321:522), Carter et al. (1992, Proc. Natl. Acad. Sci. USA 89: 4285), Sandhu (1992, Crit. Rev. Biotech. 12: 437), Singer et al. (1993, J. Immun. 150: 2844), Sudhir (ed., Antibody Engineering Protocols, Humana Press, Inc. 1995), Kelley ("Engineering Therapeutic Antibodies", in Protein Engineering: Principles and Practice Cleland et al. (eds.), pages 399-434 (John Wiley & Sons, Inc. 1996), and by Queen et al., U.S. Pat. No. 5,693,762 (1997).

### 4.2.2 Immunodiagnostic assays

The above antigen-binding molecules have utility in measuring directly or indirectly modulation of FGF signaling pathway gene expression in healthy and diseased states, through techniques such as ELISAs and Western blotting. Illustrative assay strategies which can be used to detect a target polypeptide of the invention include, but are not limited to, immunoassays involving the binding of an antigen-binding molecule to the target polypeptide (e.g., a FGF polypeptide) in the sample, and the detection of a complex comprising the antigen-binding molecule and the target polypeptide. Exemplary immunoassays are those that can measure the level or functional activity of a target molecule of the invention. Typically, an antigen-binding molecule that is immunointeractive with a target polypeptide of the invention is contacted with a biological sample suspected of containing the target polypeptide. The concentration of a complex comprising the antigen-binding molecule and the target polypeptide is measure in and the measured complex concentration is then related to the concentration of target polypeptide in the sample. Consistent with the present invention, the presence of an aberrant concentration, especially an elevated concentration, of the target polypeptide is indicative of the presence of, or probable affliction with, adipogenic dysfunction including obesity.

Any suitable technique for determining formation of an antigen-binding molecule-target antigen complex may be used. For example, an antigen-binding molecule according to the invention, having a reporter molecule associated therewith may be utilised in immunoassays. Such immunoassays include, but are not limited to, radioimmunoassays (RIAs), enzyme-linked immunosorbent assays (ELISAs) and immunochromatographic techniques (ICTs), Western blotting which are well known to those of skill in the art. For example, reference may be made to Coligan *et al.* (1994, *supra*) which discloses a variety of immunoassays that may be used in accordance with the present invention. Immunoassays may include competitive assays as understood in the art or as for example described *infra.* It will be understood that the present invention encompasses qualitative and quantitative immunoassays.

Suitable immunoassay techniques are described for example in U.S. Patent Nos. 4,016,043,4, 424,279 and 4,018,653. These include both single-site and two-site assays of the non-competitive types, as well as the traditional competitive binding assays. These assays also include direct binding of a labelled antigen-binding molecule to a target antigen.

Two site assays are particularly favoured for use in the present invention. A number of variations of these assays exist, all of which are intended to be encompassed by the present invention. Briefly, in a typical forward assay, an unlabelled antigen-binding molecule such as an unlabelled antibody is immobilised on a solid substrate and the sample to be tested brought into contact with the bound molecule. After a suitable period of incubation, for a period of time sufficient to allow formation of an antibody-antigen complex, another antigen-binding molecule, suitably a second antibody specific to the antigen, labelled with a reporter molecule capable of producing a detectable signal is then added and incubated, allowing time sufficient for the formation of another complex of antibody-antigen-labelled antibody. Any un-reacted material is washed away and the presence of the antigen is determined by observation of a signal produced by the reporter molecule. The results may be either qualitative, by simple observation of the visible signal, or may be quantitated by comparing with a control sample containing known amounts of antigen. Variations on the forward assay include a simultaneous assay, in which both sample and labelled antibody are added simultaneously to the bound antibody. These techniques are well known to those skilled in the art, including minor variations as will be readily apparent. In accordance with the present invention, the sample is one that might contain an antigen including a tissue or fluid as described above.

In the typical forward assay, a first antibody having specificity for the antigen or antigenic parts thereof is either covalently or passively bound to a solid surface. The solid surface is typically glass or a polymer, the most commonly used polymers being cellulose, polyacrylamide, nylon, polystyrene, polyvinyl chloride or polypropylene. The solid supports may be in the form of tubes, beads, discs or microplates, or any other surface suitable for conducting an immunoassay. The binding processes are well known in the art and generally consist of cross-linking, covalently binding or physically adsorbing, the polymer-antibody complex to the solid support, which is then washed in preparation for the test sample. An aliquot of the sample to be tested is then added to the solid phase complex and incubated for a period of time sufficient and under suitable conditions to allow binding of any antigen present to the antibody. Following the incubation period, the antigen-antibody complex is washed and dried and incubated with a second antibody specific for a portion of the antigen. The second antibody has generally a reporter molecule associated therewith that is used to indicate the binding of the second antibody to the antigen. The amount of labelled antibody that binds, as determined by the associated reporter molecule, is proportional to the amount of antigen bound to the immobilised first antibody.

An alternative method involves immobilising the antigen in the biological sample and then exposing the immobilised antigen to specific antibody that may or may not be labelled with a reporter molecule. Depending on the amount of target and the strength of the reporter molecule signal, a bound antigen may be detectable by direct labelling with the antibody. Alternatively, a second labelled antibody, specific to the first antibody is exposed to the target-first antibody complex to form a target-first antibody-second antibody tertiary complex. The complex is detected by the signal emitted by the reporter molecule.

From the foregoing, it will be appreciated that the reporter molecule associated with the antigen-binding molecule may include the following: (a) direct attachment of the reporter molecule to the antigen-binding molecule; (b) indirect attachment of the reporter molecule to the antigen-binding molecule; *i*.*e*., attachment of the reporter molecule to another assay reagent which subsequently binds to the antigen-binding molecule; and (c) attachment to a subsequent reaction product of the antigen-binding molecule.

The reporter molecule may be selected from a group including a chromogen, a catalyst, an enzyme, a fluorochrome, a chemiluminescent molecule, a lanthanide ion such as Europium (Eu³⁴), a radioisotope and a direct visual label.

In the case of a direct visual label, use may be made of a colloidal metallic or non-metallic particle, a dye particle, an enzyme or a substrate, an organic polymer, a latex particle, a liposome, or other vesicle containing a signal producing substance and the like.

A large number of enzymes suitable for use as reporter molecules is disclosed in United States Patent Specifications U.S. 4,366,241, U.S. 4,843,000, and U.S. 4,849,338. Suitable enzymes useful in the present invention include alkaline phosphatase, horseradish peroxidase, luciferase, β-galactosidase, glucose oxidase, lysozyme, malate dehydrogenase and the like. The enzymes may be used alone or in combination with a second enzyme that is in solution.

Suitable fluorochromes include, but are not limited to, fluorescein isothiocyanate (FITC), tetramethylrhodamine isothiocyanate (TRITC), R-Phycoerythrin (RPE), and Texas Red. Other exemplary fluorochromes include those discussed by Dower *et al.* (International Publication WO 93/06121). Reference also may be made to the fluorochromes described in U.S. Patents 5,573,909 (Singer et al), 5,326,692 (Brinkley et al). Alternatively, reference may be made to the fluorochromes described in U.S. Patent Nos. 5,227,487, 5,274,113, 5,405,975, 5,433,896, 5,442,045, 5,451,663, 5,453,517, 5,459,276, 5,516,864, 5,648,270 and 5,723,218.

In the case of an enzyme immunoassay, an enzyme is conjugated to the second antibody, generally by means of glutaraldehyde or periodates. As will be readily recognised, however, a wide variety of different conjugation techniques exist which are readily available to the skilled artisan. The substrates to be used with the specific enzymes are generally chosen for the production of, upon hydrolysis by the corresponding enzyme, a detectable colour change. Examples of suitable enzymes include those described *supra.* It is also possible to employ fluorogenic substrates, which yield a fluorescent product rather than the chromogenic substrates noted above. In all cases, the enzyme-labelled antibody is added to the first antibody-antigen complex. It is then allowed to bind, and excess reagent is washed away. A solution containing the appropriate substrate is then added to the complex of antibody-antigen-antibody. The substrate will react with the enzyme linked to the second antibody, giving a qualitative visual signal, which may be further quantitated, usually spectrophotometrically, to give an indication of the amount of antigen which was present in the sample.

Alternately, fluorescent compounds, such as fluorescein, rhodamine and the lanthanide, europium (EU), may be chemically coupled to antibodies without altering their binding capacity. When activated by illumination with light of a particular wavelength, the fluorochrome-labelled antibody adsorbs the light energy, inducing a state to excitability in the molecule, followed by emission of the light at a characteristic colour visually detectable with a light microscope. The fluorescent-labelled antibody is allowed to bind to the first antibody-antigen complex. After washing off the unbound reagent, the remaining tertiary complex is then exposed to light of an appropriate wavelength. The fluorescence observed indicates the presence of the antigen of interest. Immunofluorometric assays (IFMA) are well established in the art. However, other reporter molecules, such as radioisotope, chemiluminescent or bioluminescent molecules may also be employed.

It will be well understood that other means of testing target polypeptide (e.g., FGF or FGFR) levels are available, including, for instance, those involving testing for an altered level of FGF binding activity to a FGFR, or Western blot analysis of FGF or FGFR protein levels in tissues, cells or fluids using anti-FGF or anti-FGFR antigen-binding molecules, or assaying the amount of antigen-binding molecule of other FGF or FGFR binding partner which is not bound to a sample, and subtracting from the total amount of antigen-binding molecule or binding partner added.

### 5. Therapeutic and Prophylactic Uses

In accordance with the present invention, it is proposed that agents that antagonise the FGF signaling pathway are useful as actives for the treatment or prophylaxis of excess adipogenesis, including obesity, obesity-related conditions, lipomas and lipomatosis. It is also proposed that agents that agonise the FGF signaling pathway are useful for enhancing adipogenesis for example in cachexia and cachexia-related conditions. Such drugs can be administered to a patient either by themselves, or in pharmaceutical compositions where they are mixed with a suitable pharmaceutically acceptable carrier.

The adipogenesis-modulating agents of the present invention may be conjugated with biological targeting agents which enable their activity to be restricted to particular cell types. Such biological-targeting agents include substances which are immune-interactive with cell-specific surface antigens. For example, an agent which modulates the activity of a FGFR may be conjugated with an agent which is immuno-interactive with a preadipocyte-specific protein such as adipose differentiation related protein (ADRP). The presence of this immune-interactive conjugate confers preadipocyte-specificity to the effects of the FGFR-modulating agent.

Depending on the specific conditions being treated, the drugs may be formulated and administered systemically or locally. Techniques for formulation and administration may be found in "Remington's Pharmaceutical Sciences," Mack Publishing Co., Easton, Pa., latest edition. Suitable routes may, for example, include oral, rectal, transmucosal, or intestinal administration; parenteral delivery, including intramuscular, subcutaneous, intramedullary injections, as well as intrathecal, direct intraventricular, intravenous, intraperitoneal, intranasal, or intraocular injections. For injection, the drugs of the invention may be formulated in aqueous solutions, preferably in physiologically compatible buffers such as Hanks' solution, Ringer's solution, or physiological saline buffer. For transmucosal administration, penetrants appropriate to the barrier to be permeated are used in the formulation. Such penetrants are generally known in the art. Intra-muscular and subcutaneous injection is appropriate, for example, for administration of immunogenic compositions, vaccines and DNA vaccines.

The drugs can be formulated readily using pharmaceutically acceptable carriers well known in the art into dosages suitable for oral administration. Such carriers enable the compounds of the invention to be formulated in dosage forms such as tablets, pills, capsules, liquids, gels, syrups, slurries, suspensions and the like, for oral ingestion by a patient to be treated. These carriers may be selected from sugars, starches, cellulose and its derivatives, malt, gelatine, talc, calcium sulphate, vegetable oils, synthetic oils, polyols, alginic acid, phosphate buffered solutions, emulsifiers, isotonic saline, and pyrogen-free water.

Pharmaceutical compositions suitable for use in the present invention include compositions wherein the active ingredients are contained in an effective amount to achieve its intended purpose. The dose of drug administered to a patient should be sufficient to effect a beneficial response in the patient over time such as an enhancement or reduction in adipogenesis. The quantity of the drug(s) to be administered may depend on the subject to be treated inclusive of the age, sex, weight and general health condition thereof. In this regard, precise amounts of the drug(s) for administration will depend on the judgement of the practitioner. In determining the effective amount of the drug to be administered in the modulation of adipogenesis, the physician may evaluate tissue levels of components of the FGF signaling pathway, and degree of adiposity. In any event, those of skill in the art may readily determine suitable dosages of the drugs of the invention.

Pharmaceutical formulations for parenteral administration include aqueous solutions of the active compounds in water-soluble form. Additionally, suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions may contain substances which increase the viscosity of the suspension, such as sodium carboxymethyl cellulose, sorbitol, or dextran. Optionally, the suspension may also contain suitable stabilisers or agents which increase the solubility of the compounds to allow for the preparation of highly concentrated solutions.

Pharmaceutical preparations for oral use can be obtained by combining the active compounds with solid excipient, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as., for example, maize starch, wheat starch, rice starch, potato starch, gelatin, gum tragacanth, methyl cellulose, hydroxypropylmethyl-cellulose, sodium carboxymethylcellulose, or polyvinylpyrrolidone (PVP). If desired, disintegrating agents may be added, such as the cross-linked polyvinyl pyrrolidone, agar, or alginic acid or a salt thereof such as sodium alginate. Such compositions may be prepared by any of the methods of pharmacy but all methods include the step of bringing into association one or more drugs as described above with the carrier which constitutes one or more necessary ingredients. In general, the pharmaceutical compositions of the present invention may be manufactured in a manner that is itself known, e.g., by means of conventional mixing, dissolving, granulating, dragee-making, levigating, emulsifying, encapsulating, entrapping or lyophilising processes.

Dragee cores are provided with suitable coatings. For this purpose, concentrated sugar solutions may be used, which may optionally contain gum arabic, talc, polyvinyl pyrrolidone, carbopol gel, polyethylene glycol, or titanium dioxide, lacquer solutions, and suitable organic solvents or solvent mixtures. Dyestuffs or pigments may be added to the tablets or dragee coatings for identification or to characterise different combinations of active compound doses.

Pharmaceutical which can be used orally include push-fit capsules made of gelatin, as well as soft, sealed capsules made of gelatin and a plasticiser, such as glycerol or sorbitol. The push-fit capsules can contain the active ingredients in admixture with filler such as lactose, binders such as starches, or lubricants such as talc or magnesium stearate and, optionally, stabilisers. In soft capsules, the active compounds may be dissolved or suspended in suitable liquids, such as fatty oils, liquid paraffin, or liquid polyethylene glycols. In addition, stabilisers may be added.

Dosage forms of the drugs of the invention may also include injecting or implanting controlled releasing devices designed specifically for this purpose or other forms of implants modified to act additionally in this fashion. Controlled release of an agent of the invention may be effected by coating the same, for example, with hydrophobic polymers including acrylic resins, waxes, higher aliphatic alcohols, polylactic and polyglycolic acids and certain cellulose derivatives such as hydroxypropylmethyl cellulose. In addition, controlled release may be effected by using other polymer matrices, liposomes or microspheres.

The drugs of the invention may be provided as salts with pharmaceutically compatible counterions. Pharmaceutically compatible salts may be formed with many acids, including but not limited to hydrochloric, sulphuric, acetic, lactic, tartaric, malic, succinic, etc. Salts tend to be more soluble in aqueous or other protonic solvents that are the corresponding free base forms.

For any compound used in the method of the invention, the therapeutically effective dose can be estimated initially from cell culture assays. For example, a dose can be formulated in animal models to achieve a circulating concentration range that includes the IC50 as determined in cell culture (e.g., the concentration of a test agent, which achieves a half-maximal inhibition or enhancement in activity of a FGF or FGFR polypeptide). Such information can be used to more accurately determine useful doses in humans.

Toxicity and therapeutic efficacy of such drugs can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds that exhibit large therapeutic indices are preferred. The data obtained from these cell culture assays and animal studies can be used in formulating a range of dosage for use in human. The dosage of such compounds lies preferably within a range of circulating concentrations that include the ED50 with little or no toxicity. The dosage may vary within this range depending upon the dosage form employed and the route of administration utilised. The exact formulation, route of administration and dosage can be chosen by the individual physician in view of the patient's condition. (See for example Fingl et al., 1975, in "The Pharmacological Basis of Therapeutics", Ch. 1 pl).

Dosage amount and interval may be adjusted individually to provide plasma levels of the active agent which are sufficient to maintain FGF or FGFR-inhibitory or enhancement effects. Usual patient dosages for systemic administration range from 1-2000 mg/day, commonly from 1-250 mg/day, and typically from 10-150 mg/day. Stated in terms of patient body weight, usual dosages range from 0.02-25 mg/kg/day, commonly from 0.02-3 mg/kg/day, typically from 0.2-1.5 mg/kg/day. Stated in terms of patient body surface areas, usual dosages range from 0.5-1200 mg/m²/day, commonly from 0.5-150 mg/m²/day, typically from 5-100 mg/m²/day.

Alternately, one may administer the compound in a local rather than systemic manner, for example, *via* injection of the compound directly into a tissue, which is preferably subcutaneous or omental tissue, often in a depot or sustained release formulation.

Furthermore, one may administer the drug in a targeted drug delivery system, for example, in a liposome coated with tissue-specific antibody. The liposomes will be targeted to and taken up selectively by the tissue.

In cases of local administration or selective uptake, the effective local concentration of the agent may not be related to plasma concentration.

The present invention also contemplates a method of gene therapy of a mammal. Such a method utilises a gene therapy construct which includes an isolated polynucleotide comprising a nucleotide sequence encoding a component of the FGF signaling pathway, or a biologically active fragment thereof, wherein the polynucleotide is ligated into a gene therapy vector which provides one or more regulatory sequences that direct expression of the polynucleotide in the mammal. Typically, gene therapy vectors are derived from viral DNA sequences such as adenovirus, adenoassociated viruses, herpes-simplex viruses and retroviruses. Suitable gene therapy vectors currently available to the skilled person may be found, for example, in Robbins et al., 1998. If "anti-sense" therapy is contemplated (e.g., *Fgf*), then one or more selected portions of a *Fgf* polynucleotide may be oriented 3'→ 5' in the gene therapy vector.

Administration of the gene therapy construct to the mammal, suitably a human, may include delivery *via* direct oral intake, systemic injection, or delivery to selected tissue(s) or cells, or indirectly via delivery to cells isolated from the mammal or a compatible donor. An example of the latter approach would be stem-cell therapy, wherein isolated stem cells having potential for growth and differentiation are transected with the vector comprising a *Fgf* polynucleotide. The stem-cells are cultured for a period and then transferred to the mammal being treated.

Delivery of the gene therapy construct to cells or tissues of the mammal or the compatible donor may be facilitated by microprojectile bombardment, liposome mediated transfection (e.g., lipofectin or lipofectamine), electroporation, calcium phosphate or DEAE-dextran-mediated transfection, for example. A discussion of suitable delivery methods may be found in Chapter 9 of Ausubel *et al*., (1994-1998, *supra*).

For example, a polynucleotide encoding FGF-1 may be introduced into a cell to enhance the ability of that cell to promote adipogenesis, conversely, *Fgf-1* antisense sequences such as 3'→ 5' oligonucleotides may be introduced to decrease or impair differentiation of the cell to an adipocyte.

In an alternate embodiment, a polynucleotide encoding a modulatory agent of the invention may be used as a therapeutic or prophylactic composition in the form of a "naked DNA" composition as is known in the art. For example, an expression vector comprising the polynucleotide operably linked to a regulatory polynucleotide (e.g. a promoter, transcriptional terminator, enhancer *etc*) may be introduced into an animal, preferably a mammal, where it causes production of a modulatory agent *in vivo*, preferably in preadipocyte tissue.

The step of introducing the expression vector into a target cell or tissue will differ depending on the intended use and species, and can involve one or more of non-viral and viral vectors, cationic liposomes, retroviruses, and adenoviruses such as, for example, described in Mulligan, R.C., (1993). Such methods can include, for example:
A. Local application of the expression vector by injection (Wolff *et al.,* 1990), surgical implantation, instillation or any other means. This method can also be used in combination with local application by injection, surgical implantation, instillation or any other means, of cells responsive to the protein encoded by the expression vector so as to increase the effectiveness of that treatment. This method can also be used in combination with local application by injection, surgical implantation, instillation or any other means, of another factor or factors required for the activity of the protein.
B. General systemic delivery by injection of DNA, (Calabretta *et al*., 1993), or RNA, alone or in combination with liposome (Zhu *et al*., 1993), viral capsids or nanoparticles (Bertling *et al.,* 1991) or any other mediator of delivery. Improved targeting might be achieved by linking the polynucleotide/expression vector to a targeting molecule (the so-called "magic bullet" approach employing, for example, an antigen-binding molecule), or by local application by injection, surgical implantation or any other means, of another factor or factors required for the activity of the protein encoded by the expression vector, or of cells responsive to the protein. For example, in the case of a liposome containing antisense Fgf polynucleotides, the liposome may be targeted to MVEC by the incorporation of immuno-interactive agents into the liposome coat which are specific for MVEC-surface antigens. An example of a MVEC-specific cell surface antigen is PECAM-1.
C. Injection or implantation or delivery by any means, of cells that have been modified *ex vivo* by transfection (for example, in the presence of calcium phosphate: Chen *et al.,* 1987, or of cationic lipids and polyamines: Rose *et al*., 1991), infection, injection, electroporation (Shigekawa *et al*., 1988) or any other way so as to increase the expression of the polynucleotide in those cells. The modification can be mediated by plasmid, bacteriophage, cosmid, viral (such as adenoviral or retroviral; Mulligan, 1993; Miller, 1992; Salmons *et al*., 1993) or other vectors, or other agents of modification such as liposomes (Zhu *et al*., 1993), viral capsids or nanoparticles (Bertling *et al*., 1991), or any other mediator of modification. The use of cells as a delivery vehicle for genes or gene products has been described by Barr *et al*., 1991 and by Dhawan *et al*., 1991. Treated cells can be delivered in combination with any nutrient, growth factor, matrix or other agent that will promote their survival in the treated subject.

Embodiments of the invention are also described in the claims of the International application as filed.
1. A method for modulating adipogenesis, comprising contacting a cell with an agent for a time and under conditions sufficient to modulate a FGF signaling pathway.
2. The method of claim 1, wherein the FGF signaling pathway is selected from the FGF-1 signaling pathway and the FGF-2 signaling pathway.
3. The method of claim 1, wherein the agent modulates the expression of a gene or the level or functional activity of an expression product of the gene, wherein the gene is selected from the group consisting of a *Fgf* gene, a *Fgfr* gene, an *Hspg* gene, a gene belonging to the SHC/FRS2-RAF/MAPKKK-MAPKK-MAPK pathway, a gene belonging to the PLCγ-PKC-Ca²⁺ pathway, a gene belonging to the FGF-1 nuclear translocation pathway and a gene encoding an intracellular binding partner of a FGF.
4. The method of claim 1, wherein the cell is contacted with a agent that modulates the expression of a gene or the level or functional activity of a expression product of the gene, wherein the gene is selected from a *Fgf* gene and a gene belonging to the same regulatory or biosynthetic pathway as the *Fgf* gene.
5. The method of claim 4, wherein the cell is a microvascular endothelial cell, or precursor thereof.
6. The method of claim 4, wherein the *Fgf* gene is selected from *Fgf-1* and *Fgf-2.*
7. The method of claim 4, wherein the gene belonging to the same regulatory or biosynthetic pathway as the *Fgf* gene is selected from *P34* and *FIF.*
8. The method of claim 1, wherein the cell is contacted with an agent that modulates the expression of a gene or the level or functional activity of an expression product of the gene, wherein the gene is selected from the group consisting of a *Fgfr* gene, a gene belonging to the same regulatory or biosynthetic pathway as the *Fgfr* gene, a gene whose expression is modulated directly or indirectly by an expression product of the *Fgf* gene, or that agonises or antagonises the function of a FGFR with which a FGF interacts.
9. The method of claim 8, wherein the Fgfr gene is selected from the group consisting of *Fgfr-1*, *Fgfr-3* and *Fgfr-4.*
10. The method of claim 8, wherein the gene belonging to the same regulatory or biosynthetic pathway as the *Fgfr* gene encodes a polypeptide selected from the group consisting of syndecan-1, syndecan-2, syndecan-3, syndecan-4, glypican-1, glypican-2, glypican-3, glypican-4, glypican-5, glypican-6, perlecan, betaglycan, CFR, SHC, Crk, FRS2, Src, FAK, Nck, Shb, SHP2, GRB-2, SOS, 80K-H, pp66, Gab1, P38 MAPK, PI3K, AKT, PKB, RAS, RAF, ERK1,2, MAPKKK, MAPKK, MAPK, Jun, Fos, FPPS, PLC, Fes, PIP2, DAG, Ca²⁺ Channel, IP3, CaM kinase, PKC, PKA, cAMP, CREB and CBP.
11. The method of claim 8, wherein the gene whose expression is modulated directly or indirectly by an expression product of the *Fgf* gene is selected from the group consisting of *Pparγ, Igfbp-3*, *Igfbp-6, Igf-2, Irs-2, Pi3 kinase* and *Pkc*θ*.*
12. The method of claim 8, wherein the FGF with which the FGFR interacts is FGF-1 or FGF-1
13. The method of claim 8, wherein the cell is a preadipocyte or precursor thereof.
14. The method of claim 1, wherein the agent antagonises a FGF signaling pathway for decreasing the differentiation potential and/or proliferation of a preadipocyte.
15. The method of claim 14, wherein the agent reduces the expression of a gene or the level or functional activity of an expression product of that gene, wherein the gene is selected from the group consisting of *Fgfr-1, Fgfr-2*, *Ppar*γ, *C*/*Ebp* α, *Plo*γ*2*, *Igfbp-3*, and *Igfbp-6.*
16. The method of claim 14, wherein the agent increases the expression of a gene or the level or functional activity of an expression product of that gene, wherein the gene is selected from the group consisting of *Fgf-1, Fgfr-3*, *Igf-2, Irs-2, Pi3 kinase* and *Pkc*θ.
17. The method of claim 14, wherein the agent antagonises the function of a FGFR or interferes with the interaction between a FGFR and a FGF.
18. The method of claim 1, wherein the agent agonises a FGF signaling pathway for increasing the differentiation potential and/or proliferation of a preadipocyte.
19. The method of claim 18, wherein the agent reduces the expression of a gene or the level or functional activity of an expression product of the gene, wherein the gene is selected from the group consisting of *Fgf-1, Fgfr-3, Igf-2, Irs-2, Pi3 kinase* and *Pkcθ.*
20. The method of claim 18, wherein the agent increases the expression of a gene or the level or functional activity of an expression product of the gene, wherein the gene is selected from the group consisting of *Fgfr-1, Fgfr-2, Ppar*γ*, C*/*Ebp*α*, Plc*γ*2, Igfbp-3,* and *Igfbp-6.*
21. The method of claim 18, wherein the agent agonises the function of a FGFR or enhances, promotes or otherwise capacitates the interaction between a FGFR and a FGF.
22. The method of claim 1, wherein the agent increases or reduces the expression of the gene or the level or functional activity of an expression product of that gene by at least 10% relative to the expression, level or functional activity in the absence of the agent.
23. A method for identifying agents that modulate a FGF signaling pathway, comprising contacting a preparation with a test agent, wherein the preparation comprises (i) a polypeptide comprising an amino acid sequence corresponding to at least a biologically active fragment of a polypeptide component of the FGF signaling pathway, or to a variant or derivative thereof; or (ii) a polynucleotide comprising at least a portion of a genetic sequence that regulates the component, which is operably linked to a reporter gene; and detecting a change in the level and/or functional activity of the polypeptide component, or an expression product of the reporter gene, relative to a normal or reference level and/or functional activity in the absence of the test agent, which indicates that the agent modulates the FGF signaling pathway.
24. A method for identifying agents that modulate a FGF signaling pathway, comprising contacting a first sample of cells expressing a FGFR with a FGF and measuring at least one marker; contacting a second sample of cells expressing the FGFR with an agent and the FGF, and measuring the marker(s); and comparing the marker(s) of the first sample of cells with the marker(s) of the second sample of cells.
25. The method of claim 24, wherein the marker(s) is(are) selected from the group consisting of glycerol 3-phosphate dehydrogenase, intracellular components of the FGF pathway, and combinations thereof.
26. Use of an agent in the manufacture of a medicament for inhibiting or decreasing adipogenesis, or for controlling adipogenesis in obesity or in conditions of localised, abnormal increases in adipogenesis, wherein the agent antagonises a FGF signaling pathway.
27. The use of claim 26, wherein the agent which inhibits or otherwise decreases adipogenesis binds to a FGF or FGFR or to a genetic sequence that modulates the expression of a *Fgf* or *Fgfr* gene, as determined by: contacting a preparation comprising a FGF or FGFR polypeptide or biologically active fragment thereof, or variant or derivative of these, or a genetic sequence that modulates the expression of a *Fgf* or *Fgfr* gene; and detecting a decrease in the level or functional activity of the FGF or FGFR polypeptide or biologically active fragment thereof, or variant or derivative, or of a product expressed from the genetic sequence.
28. The use of claim 26, wherein the agent which inhibits or otherwise decreases adipogenesis antagonises a FGF signaling pathway, as determined by: contacting a FGFR and a FGF with the agent and measuring the binding of the FGFR with the FGF, whereby the agent tests positive when it reduces or abrogates the binding of the FGFR with the FGF.
29. The use of claim 28, wherein the agent is an antagonistic antigen-binding molecule specific for the FGF or the FGFR.
30. The use of claim 26, wherein the agent which inhibits or otherwise decreases adipogenesis antagonises a FGF signaling pathway, as determined by: contacting a FGFR and an HSPG with the agent and measuring the binding of the FGFR with the HSPG, , whereby the agent tests positive when it reduces or abrogates the binding of the FGFR with the HSPG.
31. The use of claim 30, wherein the agent is an antagonistic antigen-binding molecule specific for the HSPG or the FGFR.
32. The use of claim 26, wherein the agent which inhibits or otherwise decreases adipogenesis antagonises a FGF signaling pathway, as determined by: contacting a FGF and a CFR with the agent and measuring the binding of the FGF with the CFR, whereby the agent tests positive when it reduces or abrogates the binding of the FGF with the CFR.
33. The use of claim 30, wherein the agent is an antagonistic antigen-binding molecule specific for the FGF or the CFR.
34. The use of claim 26, wherein the agent which inhibits or otherwise decreases adipogenesis antagonises a FGF signaling pathway, as determined by: contacting a first sample of cells selected from preadipocytes or their precursors with a FGF and measuring differentiation and/or proliferation of the cells; contacting a second sample of cells selected from preadipocytes or their precursors with an agent and the FGF, and measuring differentiation and/or proliferation of the cells; comparing the differentiation and/or proliferation of the first sample of cells with the differentiation and/or proliferation of the second sample of cells, whereby the agent tests positive when it increases differentiation and/or proliferation of the cells.
35. The use of claim 26, wherein the agent antagonises the FGF signaling pathway by interfering with the association of the FGF and a FGFR, by interfering with the phosphorylation of a FGFR, by interfering with components of the signaling pathway upstream or downstream of the FGF/FGFR interaction, by interfering with the association of a FGFR with an HSPG, by interfering with the association of the FGF and CFR, or by interfering with the dimerisation of a FGFR. In some embodiments, agents that antagonise the FGF signaling pathway interfere with a signaling pathway selected from the TGF, IGF-1 and WNT signaling pathways.
36. The use of claim 26, wherein the agent which inhibits or otherwise decreases adipogenesis antagonises a FGF signaling pathway, as determined by: administering to an animal model, or a human, an agent that antagonises the signaling pathway and measuring the animal's responsiveness to the agent, whereby the agent tests positive when it inhibits or reduces adipogenesis in the animal.
37. Use of an agent in the manufacture of a medicament for stimulating adipogenesis in the treatment or prophylaxis of cachexia or in conditions of localised deficiencies in adiposity, wherein the agent agonises a FGF signaling pathway.
38. The use of claim 37, wherein the agent which stimulates adipogenesis binds to a FGFR or to a genetic sequence that modulates the expression of a *Fgfr* gene as determined by: contacting a preparation comprising a FGFR polypeptide or biologically active fragment thereof, or variant or derivative of these, or a genetic sequence that modulates the expression of a *Fgf* or *Fgfr* gene; and detecting an increase in the level or functional activity of the FGFR polypeptide or biologically active fragment thereof, or variant or derivative, or of a product expressed from the genetic sequence.
39. The use of claim 37, wherein the agent which stimulates adipogenesis agonises a FGF signaling pathway, as determined by: contacting a FGFR and a FGF with the agent and measuring the binding of the FGFR with the FGF, whereby the agent tests positive when it stimulates the FGFR interaction with the FGF.
40. The use of claim 39, wherein the agent is an agonistic antigen-binding molecule specific for the FGF or the FGFR.
41. The use of claim 37, wherein the agent which stimulates adipogenesis agonises a FGF signaling pathway, as determined by: contacting a FGFR and an HSPG with the agent and measuring the binding of the FGFR with the HSPG, whereby the agent tests positive when it stimulates the FGFR interaction with the HSPG,
42. The use of claim 41, wherein the agent is an agonistic antigen-binding molecule specific for the HSPG or the FGFR.
43. The use of claim 37, wherein the agent which stimulates adipogenesis agonises a FGF signaling pathway, as determined by: contacting a FGF and a CFR with the agent and measuring the binding of the FGF with the CFR, whereby the agent tests positive when it stimulates the CFR interaction with the FGF.
44. The use of claim 43, wherein the agent is an agonistic antigen-binding molecule specific for the FGF or the CFR.
45. The use of claim 37, wherein the agent which enhances adipogenesis agonises a FGF signaling pathway, as determined by: contacting a first sample of cells selected from preadipocytes or their precursors with a FGF and measuring differentiation and/or proliferation of the cells; contacting a second sample of cells selected from preadipocytes or their precursors with an agent and the FGF, and measuring differentiation and/or proliferation of the cells; comparing the differentiation and/or proliferation of the first sample of cells with the differentiation and/or proliferation of the second sample of cells, whereby the agent tests positive when it stimulates the differentiation and/or proliferation of the cells.
46. The use of claim 45, wherein the agents agonise the FGF signaling pathway by stimulating the association of the FGF with a FGFR, by stimulating the phosphorylation of a FGFR, by stimulating the association of a FGFR with an HSPG, by stimulating the association of FGF and CFR, by stimulating the dimerisation of a FGFR or by stimulating the signaling pathway upstream or downstream of the FGF/FGFR interaction.
47. The use of claim 37, wherein the agent which stimulates adipogenesis agonises a FGF signaling pathway, as determined by: administering to an animal model, or a human, an agent that agonises the signaling pathway, and measuring the animal's responsiveness to the agent, whereby the agent tests positive when it stimulates adipogenesis in the animal.
48. A method of producing an agent for modulating adipogenesis in adiposity-related conditions, comprising testing an agent suspected of modulating a FGF signaling pathway; and synthesising the agent on the basis that it tests positive for the modulation.
49. The method of claim 48, further comprising derivatising the agent, and optionally formulating the derivatised agent with a pharmaceutically acceptable carrier and/or diluent.
50. A method for detecting the presence or diagnosing the risk of an adiposity-related condition in a patient, comprising determining the presence of an aberrant gene involved in the FGF signaling pathway or of an aberrant expression product of a gene involved in the FGF signaling pathway in a biological sample obtained from the patient, wherein the aberrant gene or the aberrant expression product correlates with the presence or risk of the condition,
51. The method of claim 50, wherein the aberrant gene is selected from an aberrant *Fgf* gene and an aberrant *Fgfr* gene.
52. The method of claim 50, wherein the aberrant expression product is selected from an aberrant *Fgf* expression product and an aberrant *Fgfr* expression product.
53. A method for detecting the presence or diagnosing the risk of a condition associated with aberrantly increased adiposity in a patient, comprising determining the presence of an aberrant gene involved in the FGF signaling pathway or of an aberrant expression product of a gene involved in the FGF signaling pathway in a biological sample obtained from the patient, wherein the aberrant gene or the aberrant expression product correlates with the presence or risk of the condition.
54. The method of claim 53, wherein the condition is selected from the group consisting of obesity and conditions of localised, abnormal increases in adipogenesis
55. The method of claim 53, wherein conditions of localised, abnormal increases in adipogenesis are selected from the group consisting of lipoma and lipomatosis.
56. A method for detecting the presence or diagnosing the risk of a condition associated with aberrantly increased adiposity in a patient, comprising determining in a cell a level or functional activity of an expression product of a gene involved in the FGF signaling pathway, which is different than a normal reference level or functional activity of the expression product.
57. The method of claim 56, comprising determining an increase or elevation in the level or functional activity of an expression product of a gene selected from the group consisting of *Fgfr-1*, *Fgfr-2, Ppary, C*/*Ebpα, Plcγ2, Igfbp-3, Igfbp-6,* wherein the cell is a preadipocyte or precursor thereof.
58. The method of claim 56, comprising determining a decrease in the level or functional activity of the expression product of a gene selected from the group consisting of *Fgf-1, Fgfr-3, Igf-2, Irs-2, Pi3 kinase, Pkc*θ, wherein the cell is a preadipocyte or precursor thereof.
59. The method of claim 56, comprising determining an increase or elevation in the level or functional activity of the expression product of a gene selected from *Fgf-1* and *Fgf-2*, wherein the cell is a microvascular endothelial cell.
60. A method for inhibiting or reducing adipogenesis in obesity or in conditions of localised, abnormal increases in adipogenesis, comprising administering to a patient in need of such treatment an adipogenesis-inhibiting effective amount of an agent which impairs or interferes with a FGF signaling pathway, and optionally a pharmaceutically acceptable carrier or diluent.
61. A method for treatment or prophylaxis of cachexia or conditions of localised deficiencies in adiposity, comprising administering to a patient in need of such treatment an adipogenesis-enhancing effective amount of an agent which stimulates a FGF signaling pathway, and optionally a pharmaceutically acceptable carrier or diluent.
62. Use of an agent that modulates a FGF signaling pathway in the preparation of a medicament for treating or preventing an adiposity-related condition.

In order that the invention may be readily understood and put into practical effect, particular preferred embodiments will now be described by way of the following non-limiting example.

### EXAMPLES

### EXAMPLE 1

### Biopsy, Isolation and Culture of Human Preadipocytes and MVEC

### Materials and Methods

### Production of Anti-PECAM-1 Antibody-Coated Magnetic Beads

Dynabeads M-450 with covalently bound sheep anti-Mouse IgC1 (Dynal) are coated with purified mouse anti-human monoclonal antibody to PECAM-1 (CD31) (PharMingen) as per manufacturer's instructions. Dynabeads coated with anti-PECAM-1 antibody are resuspended and stored sterile at 4° C in deionised phosphate buffered saline (DPBS) + 0,1% BSA at a concentration of 30 mg/mL. Prepared beads remain active for at least 4 months.

### Subjects

Paired omental (O) and abdominal subcutaneous (S) adipose tissue biopsies are obtained from 4 male (average age 69 years, range 66-70 yrs; average BMI 27, range 26-29) and 5 female (average age 55 years, range 39-67 yrs; average BMI 27, range 20-32) patients undergoing elective open-abdominal surgical procedures (either gynaecological or vascular surgery). None of the patients had diabetes or severe systemic illness and none were taking medications known to affect adipose tissue mass or metabolism. The protocol was approved by the Research Ethics Committees of the Princess Alexandra Hospital and the Queensland University of Technology. All patients gave their written informed consent.

### Isolation of Stromovascular cells

With reference to Figure 2, biopsies are transported to the laboratory in Ringers solution (transport time 15 min.). Preadipocytes and microvessel endothelial cells are isolated from the same biopsies. **(1)** After removal of visible nerves, blood vessels and fibrous tissue the fat is finely minced and incubated for 1 hr at 37° C in digest solution (25 mM HEPES, 5mM glucose, 120 mM sodium chloride, 50mM potassium chloride, and 1mM calcium chloride) containing 3 mg/mL Type II collagenase and 1.5% bovine serum albumin. The ratio of digest solution to adipose tissue is 4: 1. The resultant digest material is filtered through a 250 µm mesh (Sigma) and adipocytes and free oil are separated from the stromo-vascular components by centrifugation at 250 g for 5 min at 4° C. **(2)** The stromo-vascular pellet is resuspended, washed and centrifuged in DPBS + 10% BSA (600g, 5min., 4° C). This is repeated and followed by a final wash in DPBS alone. **(3)** The resulting pellet is incubated in 0.25% trypsin containing 1 mM ethylenediamine tetraacetic acid (EDTA) (CSL, Brisbane) for 15 min at room temperature with occasional agitation. Trypsin is neutralised by addition of Hanks' balanced salt solution (HBSS) containing 5% foetal bovine serum (ICN). **(4)** Large fragments of connective tissue are removed by filtration through 100 µm mesh (Sigma). **(5)** The filtrate is centrifuged (600g, 5min, 4° C) and the pellet is resuspended and plated into 1% gelatin coated 25cm² culture flasks (Coming) in endothelial cell (EC) growth medium (M-199; ICN) containing 10% FBS; 100 IU penicillin; 100µg/mL streptomycin, 2mM L-glutamine (all ICN Biomedical Australasia); 90 µg/µL Heparin; 30 ng/mL β-endothelial cell growth factor (β-ECGF); 0.014 M HEPES; 0.15% NaHCO₃. This mixed cell population is cultured for 3-5 days at 37° C, 5%CO₂.

### Selection of microvessel endothelial cells with anti-PECAM-1 Dynabeads

Still referring to Figure 2: **(6)** After a short culture period (approx. 3 days) the cells are incubated with 0.25% trypsin/1mM EDTA for 4-5 min., followed by neutralisation of trypsin with Hank's buffered saline solution (HBSS) + 5% FBS and centrifugation. **(7)** The pelleted cells are resuspended in 1mL HBSS+5% FBS and incubated with 50 µL of anti-PECAM-1 coated Dynabeads (15min., 4° C). **(8)** The cell/bead suspension is brought to a total volume of 10mL with HBSS+5%FBS and endothelial cells are selected using a magnetic particle concentrator for 3 min. at room temperature. With the tube still in the magnet non-selected cells (preadipocytes) in the wash are transferred to a fresh tube. Endothelial cells are then washed with a further 10mL HBSS+5%FBS and reselected using the magnetic particle concentrator (3 min.). This wash/selection procedure is repeated x 5. **(9a)** Selected cells (endothelial cells) are plated onto 1% gelatin coated culture flask in EC growth medium (as above). **(9b)** Non-selected cells (preadipocytes - PA) are centrifuged and resuspended in DMEM/Ham's F12 1:1 (ICN Biomedical Australasia) containing 100 IU penicillin, 100µg/mL streptomycin, 2mM L-glutamine, and 10% FBS (PA growth medium).

### Purification of endothelial cell cultures.

Still referring to Figure 2: **(10)** Separation of endothelial cells from contaminating fibroblastic cells is achieved by treating the cultures with 0.25% trypsin/1mM EDTA (T/V) for 30-40 sec., neutralising the T/V with HBSS + 5% FCS and transferring the non-adherent endothelial cells to a 1% gelatin coated flask with EC growth medium. This trypsinisation and transfer procedure is repeated 1 or 2 times over the first two weeks of culture until homogeneous endothelial cell cultures are obtained.

### Cell Culture

Cells are maintained at 37° C in an atmosphere of 5% CO₂. The medium is changed every 2 to 3 days and cells are routinely passaged with trypsin/EDTA. Endothelial cells are maintained in gelatin-coated flasks in EC growth medium whilst preadipocytes are in uncoated culture flasks in PA growth medium. As endothelial cell numbers increase, the concentration of β-ECGF in the EC growth medium is decreased from 30ng/mL to 10ng/mL. Both endothelial cells and preadipocytes are used in experimental work between passages 2 and 4.

### Culture of other cell types

The human dermal microvascular endothelial cell line, CADMEC (Cell Applications, Ine., San Diego) (cultured under the same conditions as adipose derived primary endothelial cells), and human skin fibroblasts (obtained by punch biopsy and cultured under identical conditions as the human preadipocytes) are used as positive and negative controls, respectively, for endothelial cell studies.

### Characterisation of endothelial cells.

Microvascular endothelial cells (MVEC) obtained from adipose tissue biopsies are characterised in a number of ways.

### MORPHOLOGY

Cultures are examined by inverted phase-contrast microscopy for the characteristic cobblestone morphology of endothelial cells (Figure 3).

### IMMUNOFLUORESCENCE

Cells are evaluated by immunofluorescence using specific monoclonal antibodies for expression of von Willebrand's Factor (vWF) (Clone F8/86, DAKO) and platelet endothelial cell adhesion molecule-1 (PECAM-1; CD31) (Clone JC/70A, DAKO). Cells are grown to confluence in individual wells of 24-well plates (1% gelatin coated). Control cells (human dermal microvascular endothelial cells - CADMEC), primary cultures of human preadipocytes and human dermal fibroblasts) are processed in parallel. After removal of medium, cells are fixed in 2% paraformaldehyde (BDH Laboratory Supplies, England), 2min. at room temperature (RT). Cells are permeabilised with 0.1% Triton X100 (Ajax Chemicals, Australia), 30 sec at RT. Fixed and permeabilised cells are washed and blocked with 1% BSA in PBS (x3) prior to incubation for 4 hrs at 4° C with primary antibodies applied after dilution in PBS + 1% BSA (all antibodies are used at 1:100 dilution). To preclude false positives produced by nonspecific binding of secondary antibodies, all cell types are also treated in a similar manner with either buffer substituting for primary antibody or with non-immune antibody (iso-type control). The cells are washed with PBS (x3) then incubated at room temperature for 30 min with fluorescein isothiocyanate (FITC)-labelled secondary antibody (rabbit anti-mouse IgG FITC; DAKO) at 1:50 dilution in PBS + 1% BSA. Cells are washed (x 2) with PBS then nuclei are counter-stained with propidium iodide (stock: 5 mg propidium iodide in 100 mL 0.1M trisodium citrate; working solution: I part stock to 3 parts 0.1M PBS) for 5 min at 4 C. Cells are washed a further 2 times with PBS before being examined and photographed using a Nikon Eclipse TE300 Inverted Microscope with a Nikon TE-FM Epi-Fluorescence attachment and a Nikon F70 Camera with Kodak MAX 400 ASA film. The expression of E-selectin (CD62E) is also investigated, using a monoclonal antibody (Clone BBIG-E4, R&D Systems, Inc) and immunofluorescence as above, in cells pretreated for 4 hrs in growth medium containing 10 ng/mL tumour necrosis factor (TNF) α (Biosource International, USA). Results shown in Figure 3.

### GENE EXPRESSION.

MVEC and CADMEC are examined for expression of endothelial nitric oxide synthase (eNOS) by the NOS3 gene. Total RNA is extracted from the cells using Tri-reagent (Sigma) according to the manufacturer's instructions. Two micrograms of RNA is converted into cDNA using Expand Reverse Transcriptase (Roche) with standard methodologies. PCR is performed in a total reaction volume of 25 µL containing 1 x PCR buffer, 1 µL of cDNA, 12.5 pmols of each primer, 1.5 mM MgCl₂, and 0.625 U of Taq DNA polymerase. Primer sequences and thermal cycling conditions are as previously described (Rockett et al. In Vitro Cell Dev Biol Anim 31: 473-481 1998). PCR products are separated on 1.2% agarose gels containing 1 µg of ethidium bromide per mL in 1 x TBE buffer and viewed and photographed under ultraviolet light. φX174 markers are used.

### Characterisation of preadipocytes

Preadipocytes are characterised on the basis of morphology (phase contrast microscopy and cell counts) and differentiation capacity. The latter is assessed by G3PDH enzyme activity and triacylglycerol accumulation.

### G3PDH ACTIVITY.

Activity is assessed as previously described (Adams et al. J Clin Invest 100: 3149-53 1998) (Hutley et al in Primary Mesenchymal Cells 1st ed. Kluwer Academic 5: 173-87 2001).

### TRIACYLGLYCEROL ACCUMULATION

Cell counts and Nile Red assay are used to assess lipid accumulation.

*Cell counts.* After 14 days treatment in differentiation medium the number of lipid containing cells in each treatment is estimated under phase contrast microscopy using a 1 mm² micrometer grid (Neubauer, West Germany) at 100-fold magnification. For each treatment 10 different areas are examined and both total number of cells and percentage of lipid-containing cells are evaluated (data not shown).

*Nile Red Assay.* As previously described (Hutley *et al.* 2001 supra) preadipocytes cultured in 6-well plates are washed 3 times in phosphate buffered saline (PBS) (pH 7.4) and 150 µL of trypsin-versene is added to each well. Cells are incubated at 37°C for 10 minutes until cells detach from the culture plate. PBS containing Nile Red, at a final concentration of 1 µg/mL, is added to each well and cells are further incubated at room temperature for 5 - 7 minutes. Fluorescence is measured at room temperature in a spectrofluorometer (Aminco Bowman Series 2 Luminescence Spectrometer) at 488 nm excitation / 540 emission. Results are normalised to surface area. Each treatment is carried out in triplicate.

### EXAMPLE 2

### Effects of MVEC on Preadipocyte Proliferation and Differentiation

To investigate the role of vascular endothelial cell-derived factors on adipogenesis, the inventors examined the effects of culturing preadipocytes in vitro in the presence of growth medium containing microvascular endothelial cell-derived growth factors.

### Materials and Methods

Methods of obtaining biopsy material, isolation and culture of preadipocytes and MVEC are as per example 1.

### Preparation of conditioned medium.

Separate cultures of human adipose-derived microvascular endothelial cells (MVEC), human dermal microvascular endothelial cells (CADMEC), and human skin fibroblasts (HSP) - all at confluence on 1% gelatin coated culture ware - are each exposed to EC growth medium (see above) containing 10ng/mL β-ECGF for 48 hrs at 37°C, 5%CO₂. This medium is then collected, filtered using a 0.22µ low protein binding filter, and stored at -20°C prior to further use. EC growth medium + 10ng/mL β-ECGF is also treated as above but in culture flasks minus cells (blank control). Just prior to use each medium is thawed and a further 5% FCS is added to each.

### Preadipocyte Proliferation Assays,

Subcutaneous and omental preadipocytes and human skin fibroblasts are plated separately at about 1x10³ cells/well (subconfluent) in 96-well plates in DMEM/Ham's F12 1:1 plus 10% FCS (PA growth medium) and allowed to adhere at 37°C, 5%CO₂ for 16-20 hrs. The medium is then changed to EC growth medium which is conditioned (see above) by exposure to either confluent subcutaneous or omental MVEC, human skin fibroblasts (HSF), or wells containing no cells (blank control) (each treatment is done in quadruplicate). In separate experiments subcutaneous and omental PAs are plated as above and subsequently treated with either S MVEC, O MVEC, human dermal EC (CADMEC) conditioned media, fresh EC growth medium, or blank control. After 48 hrs, preadipocyte cell number is assessed using a formazan colorimetric assay (Promega). The water soluble tetrazolium salt 3-(4,5-dimethylthiazol-2-yl)-5-(3-carboxymethoxyphenyl)-2-(4-sulfophenyl)-2H-tetrazolium (MTS) is added to each well at a concentration of 200 µg/mL. After incubation at 37°C for 4 hrs, absorbance at 490nm is measured using a Bio-Rad 3550 microplate reader. The validity of this assay is tested in two ways; 1) preadipocytes were plated at 250; 500; 1000; 2000; 4000 cells per well (in quadruplicate) and absorbance is measured at 490 nm; 2) after measurement at A490 nm the cells are subsequently stained with propidium iodide and direct cell counts carried out using fluorescence microscopy. A total of 4 fields per well are counted and these results are compared with those obtained with formazan absorbance at 490 nm.

### Statistics

The correlation between cell number and optical density is estimated by means of Pearson's correlation coefficient. Proliferation data is evaluated by one-way analysis of variance for repeated measures. Post hoc comparison for the within condition effect is handled with paired-t tests at alpha = 0.05.

### Results

### Effect of MVEC conditioned media on PA proliferation

To determine if any soluble factors affecting preadipocyte proliferation are secreted by MVEC the human preadipocytes were exposed to 48hr treatment with MVEC conditioned medium. The results demonstrate a significant increase in the rate of proliferation of preadipocytes (both subcutaneous and omental) compared to controls (p = <0.001). This result is similar for preadipocytes treated with MVEC conditioned media from both subcutaneous (S) and omental (O) adipose tissue sites, however preadipocytes treated with 'S' MVEC show a slightly higher trend in proliferation rate than those treated with 'O' MVEC. The mitogenic effect of factors produced by adipose-derived MVEC on preadipocytes shows some specificity, as proliferation induced by human dermal MVEC (CADMEC) is not as great as that induced by adipose derived MVEC (p = 0.001). Conditioned medium from human skin fibroblasts has no increased proliferative effect on preadipocytes over the blank control. The proliferation assay in these studies was validated using known numbers of preadipocytes and results demonstrate a linear relationship between cell number and absorbance at 490nm (r² = 0.9). In a limited number of experiments direct cell count is also used to validate the results and shows a positive correlation (Pearson correlation coefficient = 0.97) with formazan absorbance at 490 nm in both test and experimental assays.

### EXAMPLE 3

### Analysis of FGF-1 Expression in Preadipocytes, Adipocytes and MVEC

Based on the observation that MVEC produce FGF-1, the investigators performed experiments to examine the role of the specific growth factor FGF-1 in the replication and differentiation of preadipocytes in vitro. The results (data not shown) reveal that preadipocytes grown in the presence of purified FGF-1 from the time of isolation show a similar, but not additive, increase in differentiation potential when compared with preadipocytes cultured in the absence of FGF-1 or other MVEC-derived factors. The investigators then designed experiments to confirm the identity of the FGF-1-producing cells, and to quantitate the FGF-1 mRNA production in the cells identified.

### Materials and Methods

Biopsies of omental and subcutaneous tissue and isolation of preadipocytes are performed as per the procedures outlined in Example 1.

### Immunofluorescent Labelling of Intracellular FGF-1

A specific anti-FGF-1 antibody (Sigma F5421) is used for the detection of intracellular FGF-1. Visualisation of labelled, intracellular FGF-1 is performed using confocal microscopy.

### Assessment of FGF-1 mRNA Expression in Preadipocytes, Adipocytes and MVEC

FGF-1 mRNA expression is assessed using real time RT-PCR. Total RNA is extracted from each cell type using a standard protocol (TRI-reagent), and cDNA is produced using the Superscript preamplification system (Life Technologies). Expression of FGF-1 is then determined using the TaqMan™ assay, a fluorescence-based real time PCR technique using the ABI Prism 7700 Sequence Detector (Perkin Elmer/Applied Biosystems).

### Quantitation of FGF-1 Protein Expression

Western blotting is then performed to assess FGF-1 protein expression in whole-cell lysates of each of the sample cell types.

### Results

Initial data show that FGF-1 mRNA and protein are expressed at very low levels in mature human adipocytes, but neither mRNA nor protein is detectable in preadipocytes. Consistent with previous results, FGF-1 mRNA and protein are both expressed at high levels by MVEC.

### EXAMPLE 4

### Characterisation of FGF-1-Induced Changes in Gene Expression

### Materials and Methods

Human omental preadipocytes are obtained by tissue biopsy from patients undergoing elective open-abdominal surgical procedures (either gynaecological or vascular surgery). None of the patients should have diabetes or severe systemic illness and none should be taking medications known to affect adipose tissue mass or metabolism. The following protocol is approved by the Research Ethics Committees of the Princess Alexandra Hospital and the Queensland University of Technology. Preadipocytes are isolated and plated according to the methods outlined in Example 1.

Preadipocytes are grown in the presence (+) or absence of (-) of human FGF-1-innoculated serum for 48 hours. Gene expression is then compared using a microarray chip, according to the manufacturer's instructions. Spots are identified on scanned microarray images using the ImaGene 4.1 (BioDiscovery) software platform. Data are interpreted using GeneSpring 4.1 software (Silicon Genetics).

Expression of phospholipase Cγ2 (PLCγ2) protein is analysed using Western blotting with immunofluorescent-labelling procedures using a monoclonal anti-PLCγ2 antibody (Santa Cruz sc-5283).

### EXAMPLE 5

### Targeting of PLCγ2 Modulators to Adipogenic Tissu

As PLCγ2 is involved in a vast number of signaling pathways in all tissues of the body, use of agents to modulate its activity for pro- or anti-adipogenic purposes requires preferential targeting of modulators to preadipocytes.

### Material and Methods

### Immunological Targeting Protocols

Monoclonal antibodies for the preadipocyte-specific protein, adipose differentiation related protein (ADRP) are raised using a standard protocol. Briefly, peptide sequences from the protein are synthesised and then used to inoculate five rabbits (in-bred albino rabbit strain) twice weekly over a period of twelve weeks. Immunological responses to the introduced peptide are monitored during this period by testing serum from the rabbits for reactivity with ADRP using *in vitro* immunocytochemical serum-based assays. The rabbits are sacrificed after twelve weeks and isolated spleen cells are cultured for the isolation and testing of anti-ADRP antibody variants. The anti-ADRP IgG antibody with the highest affinity constant is selected for conjugation with U-73122 using a carbodiimide amidation step to cross link the free carboxyl group on U-73122 to N-terminal residues on the anti-ADRP antibody.

### Lipophilic Targeting Protocols

The lipophilic benzodiazepine antagonist, flumazenil, is conjugated with U-73122 to promote the accumulation of this phospholipase inhibitor in adipose tissue. Conjugation is performed using a simple cross-linking reaction which forms a covalent bond between a selected carbon atom on each compound.

To test the anti-adipogenic potency of each conjugated U-73122 compound, three dosages of each preparation are tested in the STZ-spontaneously diabetic obese rat strain from postnatal days10 to 40. Twice-daily dosages are administered *via* intra-muscular injection. The body mass index (BMI) values of the test animals are tested daily and the rats are monitored for any adverse drug responses throughout the treatment period. The flumazenil-conjugate treatment group is closely monitored for any adverse central nervous system effects.

### EXAMPLE 6

### Expression of FGF-1 in human adipose tissue, human adipose tissue microvascular endothelial cells and murine 3T3-L1 cells

Whole cell lysates were prepared from differentiated 3T3-L1 adipocytes, adipose tissue MVEC, omental and subcutaneous human preadipocytes in the presence and absence of FGF1 from the time of isolation (over 1 week) and omental and subcutaneous isolated human adipocytes. Following protein quantitation using BCA, 20 µg of total protein was loaded per lane and proteins resolved by SDS/PAGE and transferred to nitrocellulose membrane. Protein of interest was detected using a panel of anti-FGF-1 antibodies and relevant secondary antibodies. Bound antibodies were detected using enhanced chemiluminescence.

As shown in Figure 4, FGF-1 protein was detected in 3T3-L1 cells and endothelial cells, but not detected in human preadipocytes or adipocytes under any experimental conditions. Results were consistent with all antibodies tested and confirmed by quantitative RT-PCR analysis for FGF1 mRNA (data not shown)

### EXAMPLE 7

### Effect of FGF-1, FGF-2 and IGF-1 on omental and subcutaneous preadipocyte replication and differentiation

### REPLICATION

Preadipocytes were isolated and plated in 96-well plates at 500 cells/well (sub-confluent) in serum containing medium for 12-18 hrs to allow adherence. Cells were then incubated in SCM + growth factors at 1 ng/mL for 48 hrs and a MTS proliferation assay (Promega) was performed. Results shown in Figure 5, which are presented relative to a SCM control, demonstrate marked increase in proliferation in response to both FGF-1 and FGF-2.

### DIFFERENTIATION

For differentiation experiments, preadipocytes were isolated and subcultured in endothelial cell- conditioned medium (EC-DMEM) or in the presence of growth factor for up to 2 months and then allowed to reach confluence in 6-well plates. Cells were then differentiated in serum-free, chemically modified differentiation medium including 0.1 µM Rosiglitazone. Differentiation was assessed at day 21 using a standard G3PDH assay.

The results presented in Figure 5 show that preadipocyte exposure to growth factor or adipose tissue MVEC-conditioned medium promotes subsequent differentiation under standard conditions. As with the effect on replication, FGF-1 had a more pronounced effect than FGF-2, which was, in turn, greater that the effect seen with IGF-1.

### COMBINATION FGF-1 AND FGF-2 TREATMENTS EFFECTS

Human omental and subcutaneous preadipocytes were isolated and subcultured in SCM in the presence and absence of FGF-1 or FGF-2. Upon reaching confluence, the cells were differentiated in standard chemically modified SFM + rosiglitazone in the presence and absence of FGF-1 or FGF-2. Differentiation was assessed by G3PDH activity. The results presented in Figure 6 show that both FGF-1 and FGF-2 were adipogenic if present either during replication or during differentiation. Presence throughout both processes was additive. FGF-1 had a greater adipogenic effect than FGF-2. These data suggest that the adipogenic effects of FGF-1 during replication and differentiation are independent and additive.

### EXAMPLE 8

### FGF-1 allows human preadipocytes to be differentiated in vitro in the presence of serum

A standard requirement of human preadipocyte differentiation *in vitro* is the obligatory withdrawal of serum. This contrasts with the murine adipocyte cell lines (e.g., 3T3-L1) that have high differentiation potential in SCM. It is assumed that the culture system developed for the human cells either induces down-regulation of factors necessary for differentiation, or promotes the expression of anti-differentiative factors (or both). In these experiments human omental and subcutaneous preadipocytes were isolated and subcultured in the presence of FGF-1. Cells were then differentiated in SCM plus insulin and (days 1-3) dexamethasone and rosiglitazone.

The results presented in Figure 8 show complete absence of differentiation (as evidenced by cytoplasmic lipid accumulation) in preadipocytes subcultured in SCM (A) and significant differentiation of subcutaneous (B) and omental (C) preadipocytes subcultured in SCM + FGF-1.

This is the first ever demonstration of human preadipocyte *in vitro* differentiation in the presence of serum, and provides compelling evidence for the central role of FGF-1 in human adipogenesis.

### EXAMPLE 9

### Microarray analysis of human preadipocyte gene expression by FGF-1

Total RNA was isolated from confluent subcutaneous human preadipocytes isolated and grown in either SCM (control) or SCM + FGF1, cRNA was prepared and hybridised to chips and subsequently analysed using the Affymetrix® system. Each treatment was represented by duplicate samples and two independent experiments were performed. Gene expression was considered to be influenced by FGF-1 if expression was consistently (CV<5%) increased or reduced by at least 50%. Over 100 genes fell into each category, and those currently under investigation are tabulated in Figure 9.

Up-regulation of PGFR-1 and FGFR-2 and down-regulation of FGFR-3 suggest that the FGFs-1 effect in human preadipocytes may be mediated by FGFR-1 or -2. The upregulation of peroxisome proliferator activated receptor gamma (PPARγ) and CCAAT/enhancer-binding protein alpha (C/EBPα) indicates that these key transcriptional regulators of adipogenesis are mediating the FGF-1 adipogenic effect. FGF-1 could either be promoting their expression or preventing loss of their expression in SCM (or both). Increased expression of PLCγ2 is of relevance as this is a key post-FGFR signaling molecule.

### EXAMPLE 10

### Human preadipocyte PLCγ expression is increased by FGF-1

Human preadipocytes were cultured in SCM +/- FGF-1 in 24-well plates. PLCγ expression was examined by indirect immuno-fluorescence. Non-immune primary and secondary antibody-only controls gave no staining. The results shown in Figure 10 demonstrate that expression of this molecule is increased in human PAs grown to confluence in the presence of FGF-1 as compared to cells in SCM alone. These results also show that PLC γ2 expression is greatly upregulated at confluence - the stage at which induction of differentiation occurs.

### EXAMPLE 11

### Inhibition of PLCγ impairs FGF1-induced human adipogenesi

Human subcutaneous preadipocytes were isolated and subcultured in SCM in the presence and absence of FGF-1 with and without the PLC inhibitor U-73122 (Calbiochem). Cells were then allowed to reach confluence and differentiated using the standard chemically-modified SFM including rosiglitazone in the presence and absence of FGF1 with and without U-73122. Differentiation was assessed by G3PDH activity. The results presented in Figure 11 show that U-73122 significantly impaired FGF-1-induced differentiation during the replication phase or the differentiation phase and that it also had an additive effect during both processes.

### EXAMPLE 12

### Neutralising anti-FGF-1 antibody abrogates FGF-1-induced human preadipocyte replication

Human subcutaneous preadipocytes were isolated and cultured in SCM with FGF-1 +/anti-FGF-1 antibody. Replication was assessed as outlined above. The results presented in Figure 12 show a dose-dependent reduction in replication with the antibody. These data support the efficacy of extra-cellular FGF-1-reduction strategies.

### EXAMPLE 13

### Effect on preadipocyte differentiation of inhibition of post-FGFR signaling

Human preadipocytes were isolated and subcultured in SCM + FGF1. For one week prior to differentiation, tyrosine kinase inhibitors were added to the medium. The cells were then differentiated in SFM + rosiglitazone + FGF1 +/- the inhibitors for the first 3 days. Cells were harvested on day 15 and differentiation assessed by G3PDH activity.

The compounds used for these experiments were as follows: (1) Calphostin C (Cal C) - PKC inhibitor; (2) PD 98059 (PD) - MEK inhibitor; (3) Ly 294002 (LY) - PI3-K inhibitor; (4) SB 202190 (SB 190) - p38 kinase inhibitor; and (5) SB 202474 (SB 474) - control compound for SB 190.

The results presented in Figure 13 demonstrate that inhibition of post FGFR signal transduction pathways has marked effects on FGF-1-mediated human adipogenesis. Inhibition of PKC, PI3K and PLCγ (shown above) all significantly reduce differentiation. MEK and p38 kinase inhibition during preadipocyte replication phase alone significantly reduces subsequent differentiation.

The disclosure of every patent, patent application, and publication cited herein is hereby incorporated herein by reference in its entirety.

The citation of any reference herein should not be construed as an admission that such reference is available as "Prior Art" to the instant application.

Throughout the specification the aim has been to describe the preferred embodiments of the invention without limiting the invention to any one embodiment or specific collection of features. Those of skill in the art will therefore appreciate that, in light of the instant disclosure, various modifications and changes can be made in the particular embodiments exemplified without departing from the scope of the present invention. All such modifications and changes are intended to be included within the scope of the appended claims.

## Claims

1. An agent which antagonises FGF signaling for use in treating or preventing an adiposity-related condition, wherein the agent decreases the expression of an FGF gene or the level or functional activity of an expression product of an FGF gene, thereby inhibiting or decreasing adipogenesis.

2. The agent of Claim 1, wherein the FGF is selected from the group consisting of FGF-1 and FGF-2.

3. The agent of Claim 1, wherein the agent decreases the differentiation potential and/or proliferation of a preadipocyte or preadipocyte precursor.

4. The agent of any one of Claims 1 to 3, wherein the agent antagonises the function of an FGFR with which an FGF interacts.

5. The agent of Claim 1, wherein the agent decreases expression or activity of the expression product of an FGF gene in microvascular endothelial cells (MVECs).

6. The agent of Claim 1, wherein the agent which inhibits or otherwise decreases adipogenesis binds to an FGF or to a genetic sequence that modulates the expression of an FGF gene, as determined by contacting a preparation comprising an FGF polypeptide or biologically active fragment thereof, or variant or derivative thereof, or a genetic sequence that modulates the expression of an FGF gene with the agent and detecting a decrease in the level or functional activity of the FGF polypeptide or biologically active fragment thereof, or variant or derivative, or a product expressed from the genetic sequence.

7. The agent of Claim 6, wherein the agent is an antagonistic antigen-binding molecule specific for the FGF polypeptide or is an antisense nucleic acid molecule which reduces expression of` the FGF genetic sequence.

8. The agent of Claim 7, wherein the agent reduces the expression of the Fgf gene or the level or functional activity of the FGF polypeptide by at least 10% relative to the expression, level or functional activity in the absence of the agent.

9. A method for identifying agents useful for treating or preventing an adiposity-related condition comprising contacting a preparation with a test agent, wherein the preparation comprises (i) a polypeptide comprising an amino acid sequence corresponding to at least a biologically active fragment of an FGF polypeptide; or (ii) a polynucleotide comprising at least a portion of a genetic sequence that regulates the FGF polypeptide, which is operably linked to a reporter gene; and detecting a change in the level and/or functional activity of the FGF polypeptide, or an expression product of the reporter gene, relative to a normal or reference level and/or functional activity in the absence of the test agent, wherein the change indicates that the agent antagonises the FGF signaling pathway.

10. A method of producing an agent for treating or preventing an adiposity-related condition comprising testing an agent for its ability to antagonise FGF signaling by decreasing the functional activity of an expression product of an FGF gene or decreasing expression of an FGF gene and then synthesising an agent on the basis that it antagonises FGF signaling.

11. The method of Claim 10, wherein the FGF signaling is *via* FGF- or FGF-2.

12. A method for detecting the presence or diagnosing the risk of an adiposity-related condition in a patient, comprising determining the presence of an aberrant Fgf gene or of an aberrant expression product of an Fgf gene in a biological sample obtained from the patient, wherein the aberrant expression product or aberrant gene stimulates FGF signaling, wherein the aberrant Fgf gene or aberrant expression product correlates with presence or risk of the condition.

13. The method of Claim 12, wherein the adiposity-related condition is a condition associated with aberrantly increased adiposity.

14. The method of Claim 13, wherein the condition is selected from the group consisting of obesity and localised abnormal increased adipogenesis, preferably the localised abnormal increased adipogenesis is lipoma or lipomatosis.

15. A method for detecting the presence or diagnosing the risk of a condition associated with aberrantly increased adiposity in a patient, comprising determining in a cell the level or functional activity of an expression product of an Fgf gene which is different than a normal reference level or functional activity of the expression product.

16. The method of any one of Claims 12 to 15 wherein the FGF is FGF-1 or FGF-2.

17. The agent of any one of Claims 1 to 8 or the method of any one of Claims 9 to 11, 15 or 16, wherein the condition is selected from the group consisting of obesity and localised abnormal increased adipogenesis.
